(19) Europäisches Patentamt
European Patent Office
Office européen des brevets

(11) **EP 3 585 248 B1**

(12) **EUROPEAN PATENT SPECIFICATION**

(45) Date of publication and mention
of the grant of the patent:
**15.11.2023 Bulletin 2023/46**

(21) Application number: **18704576.0**

(22) Date of filing: **19.02.2018**

(51) International Patent Classification (IPC):
*A61B 5/00* (2006.01)        *A61B 5/055* (2006.01)
*G01R 33/56* (2006.01)        *G01R 33/563* (2006.01)

(52) Cooperative Patent Classification (CPC):
**A61B 5/055; A61B 5/7264; G01R 33/56341;**
G01R 33/5608; G16H 50/20

(86) International application number:
**PCT/EP2018/054031**

(87) International publication number:
**WO 2018/153814 (30.08.2018 Gazette 2018/35)**

(54) **METHOD AND DEVICE FOR NON-INVASIVELY CLASSIFYING A TUMOROUS MODIFICATION OF A TISSUE**

VERFAHREN UND VORRICHTUNG ZUR NICHT-INVASIVEN KLASSIFIKATION TUMORARTIGER GEWEBEVERÄNDERUNGEN

PROCÉDÉ ET DISPOSITIF POUR LA CLASSIFICATION NON-INVASIVE DES MODIFICATIONS TISSULAIRES TUMORALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2017 EP 17157377**

(43) Date of publication of application:
**01.01.2020 Bulletin 2020/01**

(73) Proprietor: **Deutsches Krebsforschungszentrum
69120 Heidelberg (DE)**

(72) Inventors:
• **BICKELHAUPT, Sebastian
69126 Heidelberg (DE)**
• **KÖNIG, Franziska
69168 Wiesloch (DE)**

(74) Representative: **Altmann Stößel Dick
Patentanwälte PartG mbB
Theodor-Heuss-Anlage 2
68165 Mannheim (DE)**

(56) References cited:
• **MAKI HIRANO ET AL: "Diffusion-Weighted Imaging of Breast Masses: Comparison of Diagnostic Performance Using Various Apparent Diffusion Coefficient Parameters", AMERICAN JOURNAL OF ROENTGENOLOGY, vol. 198, no. 3, 1 March 2012 (2012-03-01) , pages 717-722, XP055401656, US ISSN: 0361-803X, DOI: 10.2214/AJR.11.7093**
• **PAUL JAEGER ET AL: "Revealing Hidden Potentials of q-Space Imaging in Breast Cancer", ARXIV.ORG, CORNELL UNIVERSITY LIBRARY, 201 OLIN LIBRARY CORNELL UNIVERSITY ITHACA, NY 14853, 27 February 2017 (2017-02-27), XP080748793,**
• **PEREZ ILEANA MONTOYA ET AL: "Diffusion weighted imaging of prostate cancer: Prediction of cancer using texture features from parametric maps of the monoexponential and kurtosis functions", 2016 SIXTH INTERNATIONAL CONFERENCE ON IMAGE PROCESSING THEORY, TOOLS AND APPLICATIONS (IPTA), IEEE, 12 December 2016 (2016-12-12), pages 1-6, XP033043788, DOI: 10.1109/IPTA.2016.7820993**

**(Cont. next page)**

EP 3 585 248 B1

• VALERIO MARIACRISTINA ET AL: "3T multiparametric MRI of the prostate: Does intravoxel incoherent motion diffusion imaging have a role in the detection and stratification of prostate cancer in the peripheral zone?", EUROPEAN JOURNAL OF RADIOLOGY, ELSEVIER SCIENCE, NL, vol. 85, no. 4, 20 January 2016 (2016-01-20), pages 790-794, XP029461280, ISSN: 0720-048X, DOI: 10.1016/J.EJRAD.2016.01.006

**Description**

Technical field of the invention

The invention relates to a method and a device for non-invasively classifying a tumorous modification of a tissue, in particular of a human tissue, preferably without an administration of contrast agents or ionizing irradiation. The method and the device according to the present invention specifically may be used in the field of oncologic imaging. However, other applications are possible.

Related art

Tissue characterization in the field of oncologic imaging by using non-invasive imaging modalities is still a challenging task. Modalities which are currently available for detecting and characterizing suspicious changes of a tissue include ultrasound, x-ray imaging, computer tomography, positron emission tomography (PET), and magnetic resonance imaging (MRI), whereby MRI is increasingly used in oncologic imaging [1]. Using MRI for detecting and characterizing suspicious change of a tissue commonly includes sophisticated examination protocols as well as an intravenous application of a contrast agent, in particular, a compound comprising gadolinium, which may, however, imply risks for allergic reactions, nephrogenic systemic fibrosis, and gadolinium deposition in the brain [2-7].

In addition, invasive biopsy is still required in a number of cases in order to gain tissue probes for histopathological analyses, such as in breast MRI [8, 9] and prostate MRI [10]. The reason for this observation is based on findings that many lesions in a tissue can only be properly characterized by using histopathological specimens as results from oncologic imaging often, still, remain relatively unspecific. As a result, many false positive findings which cause invasive procedures in healthy persons still occur. On the other hand, anxiety, stress and potential side-effects, such as bleeding, scars, or fistulas, could be prevented if a biopsy and a subsequent histopathological analysis could be avoided.

A new approach applies MRI sequences configured for mapping water diffusion [11]. This approach is based on the assumption that water diffusion is related to tissue properties. By way of example, water has been considered not to move as freely in malignant tissue as in benign tissue as a result of a restricted possibility of diffusion between densely packed cell conglomerates. Using diffusion weighted imaging (DWI) in connection with a proper setting of the corresponding MRI sequences may, thus, be advantageous in characterizing tissue changes by means of non-invasive imaging.

In addition, an in-depth analysis of DWI images might be used for further insights into tissue properties which are correlated with histopathology. By applying different fitting models, DWI has been reported to allow assessing microstructural tissue properties which correlate to isolated aspects of the tissue, including tissue cellularity, such as indicated by an apparent diffusion coefficient (ADC), or tissue integrity, such as indicated by Kurtosis [12-16]. However, known quantification schemes which are used for microstructural DWI correlates are, in general, be considered as individual approaches which are directed to tissue characterization by using absolute quantification values of solitary parameters for each quantification scheme. As a result, the quantification schemes parameters obtained in this fashion proved to be of limited diagnostic value and, usually, required a further invasive biopsy.

A similar approach [17] is provided by a mathematical model which characterizes water diffusion in vascular, EES, and intracellular compartments in tumors. For this purpose, a sum of three parametric models is calculated, wherein each parametric model describes a diffusion magnetic resonance signal in a separate population of water from one of the three components, wherein a first signal arises from intracellular water trapped inside cells while a second signal arises from EES water adjacent to, but outside cells and blood vessels and a third signal arises from water in blood undergoing microcirculation in a capillary network. This model does not incorporate an exchange between the three water populations, thus, each quantification scheme is treated individually.

In a further approach [18] DWI of prostate and/or bladder cancer patients scheduled for radical prostatectomy were acquired and used to compute the apparent diffusion coefficient (ADC), an intravoxel incoherent motion (IVIM: the pure diffusion coefficient $D_t$, the pseudo-diffusion fraction $F_p$ and the pseudo-diffusion coefficient $D_p$), and high b-value parameters within the index lesion. These parameters were, subsequently, used in a separate fashion or combined in a logistic regression model in order to differentiate lesions. In a similar manner, using a fractional order calculus model (FROC) in brain imaging was evaluated using an imaging protocol with more than 10 b-values to extract two parameters (D as an approximation of the apparent diffusion coefficient and $\beta$) which, separately or combined with a logistic regression model, was used for differentiating high grade from low grade brain lesions [19].

A similar method for prostate cancer detection is presented in [20] which is based on texture features which are extracted from a grid that is placed on diffusion weighted imaging (DWI) parametric maps, in particular, parametric maps from monoexponential AOCm, kurtosis AOCk and K. Hereinafter, the texture maps were divided in cubes, and median texture features were calculated for each cube. The obtained features were used to train prediction models, wherein an *area under the curve* (AUC) value was used to assess the prediction efficiency. In total, 875 texture features were extracted in this manner using Gabor filter, GLCM, LBP, Haar transform, and Hu moments. In addition, statistical features were calculated. As a result, AUC values of 0.81 to 0.85 could be demonstrated.

Problem to be solved

It is therefore an objective of the present invention to provide a method and a device for non-invasively classifying a tumorous modification of a tissue, in particular of a human tissue, which at least partially avoid the disadvantages of known methods and devices.

Hereby, it is a particular objective of the present invention to allow comprehensively characterizing different microstructural properties of the tissue by using a single non-invasive, contrast-agent free, and radiation-free examination for gaining insight in suspicious microstructural changes of the tissue in a highly accurate and fast manner, which may especially be adapted for use in clinical routine.

Summary of the invention

This problem is solved by a method and a device for non-invasively classifying a tumorous modification of a tissue, in particular of a human tissue, according to the subject-matter of the independent claims. Preferred embodiments of the invention, which may be realized in an isolated way or in any arbitrary combination, are disclosed in the dependent claims.

As used in the present specification, the term "comprising" or grammatical variations thereof, are to be taken to specify the presence of stated features, integers, steps or components or groups thereof, but do not preclude the presence or addition of one or more other features, integers, steps, components or groups thereof. The same applies to the term "having" or grammatical variations thereof, which is used as a synonym for the term "comprising".

In a first aspect of the present invention, a computer-implemented method for non-invasively classifying a tumorous modification of a tissue, preferably a tissue provided by an animal, more preferably by a mammal, and, most preferably by a human, into one of at least two classes, wherein each class refers to a different stage of the tumorous modification, is disclosed. Herein, the method according to the present invention comprises at least the following steps a) to step e), wherein, however, additional steps may further be performed. In one embodiment, steps a) to e) may be performed in a sequential approach, commencing with step a), continuing with steps b), c) and d) in this order, and finishing with step e), wherein, however, a subsequent step may at least partially be performed concurrently with a previous step. In an alternative embodiment, the mentioned steps may be performed in an integrative approach or in a mixed approach combining the sequential approach and the integrative approach, in particular, for minimizing time and/or storing space required for performing the present method.

In particular, the method comprises the steps of:

a) receiving raw magnetic resonance imaging (MRI) data that has been recorded by applying at least one diffusion weighted imaging (DWI) sequence using three to nine different b-values to a tissue being suspicious to a tumorous modification without application of a contrast agent;
b) extracting at least two quantification scheme parameters from the raw MRI data by using at least one quantification scheme, wherein each of the quantification scheme parameters is related to a microstructural property of the tissue;
c) applying a weight to each quantification scheme parameter, wherein the weight is dependent on a kind of the tissue and on the quantification scheme, whereby a set of weighted quantification scheme parameters is obtained;
d) determining a scoring value by combining the weighted quantification scheme parameters within the set, wherein each of the weighted quantification scheme parameters is used only once for determining the scoring value; and
e) classifying the tumorous modification of the tissue into one of at least two classes according to the scoring value.

Thus, the present method refers to a characterization of tissues by applying non-invasive imaging modalities. As generally used, the term "tissue" refers to a partition of an animal body, preferably of a mammal body, and, more preferred of a human body, which comprises an ensemble of similar cells having a similar origin and which are assembled together to jointly performing a particular function in the body. Consequently, the tissue can be considered as a cellular organization which is arranged at an intermediate level between a single cell and a complete organ, wherein the organ can be considered as being formed by functionally arranging a plurality of tissues.

In general, an invasive study of tissues is known as "histology" or, in connection with disease, as "histopathology". As described below in more detail, histopathological analyses have been used in order to demonstrate that results obtained for the tissues by the non-invasive characterization according to the present invention confirm with actual findings in the suspicious tissues.

In addition, a particular histopathological analysis may, preferably, be applied in order to provide a training data set, wherein the term "training data set" refers to a set of data which comprises data being determined according to the method as described herein that have been confirmed by histopathological analysis. Consequently, the training data set may, preferably, be applied in order to determine the respective weights to be applied for a particular kind of the tissue and a selected quantification scheme.

In contrast hereto, the term "non-invasive" refers to an *in vivo* method of studying one or more tissues, wherein the tissue under investigation is able to remain in the body during the study and does, herein, not receive any treatment by a contrast agent, such as by a gadolinium-comprising compound. Thus, in contrast to a histopathological analysis, a non-invasive method allows determining one or more properties of the tissue without removing it from the body of a person or an animal. As mentioned above, the non-invasive methods, in particular, include ultrasound, x-ray imaging, computer tomography, positron emission tomography (PET), and magnetic resonance imaging (MRI), whereby MRI is used as the preferred non-invasive method according to the present invention.

As further mentioned above, the preferred non-invasive method for characterizing tissue changes comprises diffusion weighted imaging (DWI) in connection with a proper setting of MRI sequences which are, especially, configured for mapping water diffusion. This approach reflects that water diffusion can reasonably be assumed to be related to those tissue properties which appear relevant with regard to a tumorous modification of the tissue. As generally used, the term "tumorous modification" refers to a change of a particular tissue which can be attributed to a presence of a tumor in the particular tissue. Since the presence of a tumor tends to reorganize the cells in the particular tissue, such as, directed to generating more densely packed cell conglomerates, a movement of water molecules may be slightly impaired by the presence of a tumor in the particular tissue. Consequently, diffusion may slightly be impaired between the cell conglomerates, thus, resulting in a decrease of diffusion-related properties of the particular tissue.

The tumorous modification can, in general, be assigned to a presence of cancer within the tissue. The term "cancer" in the context of this invention refers to a disease of an animal, in particular of a mammal, and, especially of a human, which is characterized by an uncontrolled growth by a group of body cells, usually denoted as "cancer cells". This uncontrolled growth may be accompanied by intrusion into and destruction of surrounding tissue (i.e. "invasion") and possibly spread of cancer cells to other locations in the body (i.e. "metastasis"). Preferably, the cancer may be selected from the list consisting of: acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor.

In particular, the tumorous modification which can, preferably, be classified according to the present invention may be selected from the group consisting of female breast cancer and cervical cancer and male prostate cancer. Corresponding results which have been achieved by respectively modified tissues are illustrated below. However, the present invention may also be applicable to other kinds of tumorous modification, such as a tumorous modification which may be due to one or more of the above-mentioned types of cancer.

According to the present invention, the tumorous modification of a tissue is classified in a manner that it is, according to the properties of the tumorous modification in relation to the scope of the selected classes, sorted into one of at least two classes. Herein, the term "class" refers to a different stage of the tumorous modification which may be selected depending on a particular kind of tumorous modification, wherein the term "stage" refers to a particular grade to which specific a cancer can be assigned to. Preferably, each class comprises a scope which may be defined in a fashion that the classification leads to a single defined result. By way of example, the tumorous modification may be sorted into one of the two classes benign or malignant, wherein the term "malignant" relates to a particular tissue modification which is not self-limited in growth, capable of invading into adjacent tissues, and capable of spreading to distant tissues while the term "benign" refers to a particular tissue modification which does not comprise any of the mentioned properties of the malignant stage. Alternatively, the tumorous modification may be sorted into one of the three classes benign, clinically insignificant, or clinically significant, wherein the term "benign" is defined as above, the term "clinically insignificant" refers to a malignant stage which is, however, not considered as being subject to surgical intervention while the term "clinically significant" refers to a malignant stage which is considered as being subject to surgical intervention. However, especially depending on the particular kind of tumorous modification, further kinds of classes may also be feasible for classifying the tumorous modification of a tissue according to the purposes of the present invention.

Further, the method according to the present invention is a computer-implemented method. As generally used, the term "computer-implemented method" refers to a method which involves a programmable apparatus, in particular, a computer, a computer network, or a readable medium carrying a program, whereby one or more of the features of the invention are preformed by means of at least one program. With particular regard to the present invention, the present method is, thus, being performed on a programmable apparatus which is configured for this purpose, such as by providing a particular computer program. As a result, the present method may, as demonstrated below in more detail, particularly affect the efficiency of classifying the tumorous modification, thereby providing highly reliable results based on a non-invasively investigation and evaluation of the of the tissue, thus, avoiding false positive findings which may result in invasive procedures in healthy persons as well as anxiety, stress and potential side-effects, such as bleeding, scars, or fistulas, due to a biopsy.

According to step a), raw magnetic resonance imaging (MRI) data are received. As generally used, the terms "magnetic resonance imaging" or "MRI" refer to a process which is, in particular, used for obtaining images ("MRI images") of an animal body, preferably of a mammal body, and, more preferred of a human body, or a partition thereof, such as a particular organ or tissue, in both health and disease, wherein the desired MRI images are generated by applying a magnetic resonance imaging device ("MRI device") being configured for providing strong magnetic fields, magnetic field gradients and electromagnetic waves in the radiofrequency spectral range. For this purpose, the raw data may be acquired by using a known MRI device and a strength of the magnetic field of 1 T to 7 T, such as 1.5 T or 3 T. Herein, the animal body, preferably the mammal body, and, more preferred, the human body, or a partition thereof, such as the particular organ or tissue, has not been treated by application of any contrast agent, such as a gadolinium-comprising compound, prior to or concurrently with acquiring the raw MRI data used for obtaining the desired MRI images.

With particular regard to the present invention, the raw data have been recorded by applying at least one diffusion weighted imaging (DWI) sequence using three to nine different b-values, to the tissue which may be considered as suspicious to a tumorous modification. In general, diffusion may be considered as a further relaxation mechanism in addition to the known T1 and T2 mechanisms in MRI. As already mentioned above, the diffusion weighted imaging (DWI) sequences are MRI sequences which are, especially, configured for mapping water diffusion in the tissue being investigated. As generally used, the term "MRI sequence" refers to a predefined succession of radiofrequency pulses and related magnetic field gradients, wherein the succession and the particular parameters with respect to each radiofrequency pulse and each related magnetic field gradient are configured in order to provide at least one particular MRI image which is, especially, configured for a particular purpose, wherein the particular purpose is related to diffusion weighting in the case of the present invention. By way of example, DWI sequences can be provided by using technical equipment associated to the MRI device, such as coils with a particular numbers of channels. The raw data can be acquired by employing a particular DWI sequence, such as epiDWI, resolveDWI, or DWIBS. However, other kinds of sequences may also be feasible. Herein, a particular setting may be used for the selected DWI sequence which may be adjusted to a slice thickness, a fat saturation or to a different parameter. However, other kinds of DWI sequence may also be feasible. As used herein, the term "raw data", thus, refers to primary data which are provided by the MRI device, such as a particular MRI image or at least one specific parameter which is related to the particular MRI image, whereby the parameters of the selected DWI sequence and the related particular setting are, additionally taken into account. Hereby, the raw data has, in general, not been subjected to processing by software designated for such a purpose.

A specific parameter which is used in recording a particular DWI sequence is a so-called "b-value". Herein, the term "b-value" refers to a factor which is correlated with a magnetic field gradient being used for generating at least one DWI, whereby a higher b-value, in general, correlates with stronger diffusion effects.

Using the 'traditional monoexponential model' as a quantification scheme, a signal S which may be obtained after applying a diffusion gradient to a tissue may, specifically, be estimated according to Equation (1) as

$$S = S_0 \cdot e^{(-bD_A)}, \qquad\qquad (1)$$

wherein $S_0$ is a baseline magnetic resonance signal, $D_A$ a diffusion coefficient also denoted as "ADC" in the "traditional monoexponential model" quantification scheme, and b the related b-value. By selecting a particular b-value prior to imaging, the degree of diffusion weighting may, thus, be, chosen. Since the term $e^{-bD}$ in Equation (1) is a dimensionless number, the unit for $b$ is the inverse of the unit for $D_A$. As a result, $b$ is expressed as value of time per area. For further details concerning the b-value, reference may, for example, be made at www.mriquestions.com/what-is-the-b-value.html (as viewed on February 17, 2017).

Alternatively or in addition, the b-value may be determined in a quantification scheme based on 'diffusional kurtosis imaging' (DKI) according to Equation (2) as

$$S = S_0 \cdot e^{\left(-bD_K + \frac{1}{6}b^2 D_K^2 K_K\right)}, \qquad (2)$$

wherein $S_0$ is the baseline magnetic resonance signal, $D_K$ or AKC the diffusion, $K_K$ the kurtosis coefficient in the DKI quantification scheme, and $b$ the related b-value. By using a different quantification scheme, such as one of the further quantification schemes as mentioned below in more detail, a different relationship for the b-value may be applicable.

In the present invention, the at least one DWI sequence is obtained by using three to nine different b-values, more preferred three to six different b-values, specifically three to four different b-values. Herein, the number of b-values can be used either by applying three to nine different DWI sequences, each DWI sequence having one of the different b-values or by simulating and/or determining a final number of three to nine b-values out of a lower or higher number of b-values as acquired in a manner familiar in the art. Herein, the higher number of b-values may also include zero or void measurements, in which the DWI acquisition might be expanded by an additional b-value or other type of acquisition without the dedicated need for calculating DWI quantification schemes and its parameters, for example, a particular sequence may be repeated with the same b-values or a considerably similar b-values differing by less than 50 s/ mm$^2$, such as by less than 10 s/ mm$^2$, such as by only 1-2 s/ mm$^2$, thus, delivering virtually the same result. By way of example, three different b-values may be set to 0, to 750 s/mm$^2$, and to 1500 s/mm$^2$, or the four different b-values may be set to 0, to 50 s/mm$^2$, to 500 s/mm$^2$, and to 1500 s/mm$^2$. Herein, two adjacent b-values may, preferably, be separated from each other by at least 50 s/ mm$^2$. However, other kinds of b-values, in particular of 0 to 4000 s/mm$^2$, may also be chosen for the purpose of recording diffusion properties of the tissue under investigation which may allow obtaining quantitative raw data which can, subsequently, be used for comprehensively characterizing the tissue properties. In accordance with the objective of the present invention, it may, however, be advantageous to keep the number of b-values and, thus, the number of consecutively applied DWI sequences as low as possible, in particular, in order to achieve an examination time as short as possible for performing step a) in order to qualify the present method for use in clinical routine.

As a result, the raw MRI data are received for the at least one DWI sequence for further processing in the subsequent step b). As generally used, the term "receiving data" refers to a process of obtaining data, such the MRI raw data, by the programmable apparatus used for performing the method steps of the present invention, wherein the raw data are stored and prepared for the further processing. In addition, pre-processing steps, such as removing outliers, may also be applied to the raw data prior to further processing in the subsequently described step b).

According to step b), two to four quantification schemes, are used for extracting at least two quantification scheme parameters $\{k_i, p_i; i = 1 \ldots n, n \geq 2\}$, preferably two to twenty quantification scheme parameters, more preferred two to ten quantification scheme parameters, from the raw MRI data. Herein, the quantification scheme is selected from 'diffusional kurtosis imaging' (DKI), "traditional monoexponential model', 'intravoxel incoherent motions' (IVIM), or 'fractional order calculus' (FROC), whereas the quantification scheme parameter may, preferably, be selected from ADC; AKC; D-IVIM, or f-IVIM. It may be emphasized here that the "traditional monoexponential model' is often also denoted by the same term "ADC" which, strictly speaking, denotes the corresponding quantification scheme parameter obtained by using the traditional monoexponential model as described above in more detail. By way of example, a first quantification scheme parameter ADC or $D_A$ may, preferably, be extracted from Equation (1) while a second quantification scheme parameter diffusion kurtosis coefficient $D_K$ or AKC may, preferably, be extracted from Equation (2). However using other kinds of quantification schemes or other corresponding quantification scheme parameters may also be feasible.

As used herein, the term "quantification scheme" refers to a process of generating quantified information from the raw MRI data as received by applying the at least one DWI sequence in accordance with step a). In other words, generating the quantified information includes a step of transformation of the raw data by using the at least one quantification schemes for the DWI sequence. Since the quantified information is obtained by using the DWI sequence and DWI has been reported to allow assessing microstructural tissue properties, the quantified information, i.e. each of the quantification scheme parameters, which may be generated by applying the at least one quantification scheme is, therefore, related to a microstructural property of the tissue considered as suspicious to a tumorous modification. By way of example, the tissue might be characterized by using a first quantification scheme and a second quantification scheme, the first, the second, and a third quantification scheme, or the first, the second, the third, and a fourth quantification scheme in any combination.

As further used herein, the term "extracting" refers to applying the selected number of quantification schemes by using any process which is known in the art as being suitable for a quantification of DWI sequences, namely in the context of the claimed invention 'diffusional kurtosis imaging' (DKI), "traditional monoexponential model', 'intravoxel incoherent motions' (IVIM), or 'fractional order calculus' (FROC). Herein DKI, in general, takes into account a non-Gaussianity of a distribution. While the traditional monoexponential model assumes a monoexponential decay of the diffusion signal, IVIM takes into account that flowing blood may contribute to the diffusion signal by employing a biexponential model, wherein a faster decaying exponential may be separable from a slower exponential decay reflecting true water diffusion. Further, the FROC analysis is adapted to determine a tissue heterogeneity in detected lesions within the tissue.

In the context of the claimed invention, a fitting procedure is applied for quantitatively determining the desired quantification scheme parameter from the applied quantification scheme. However, only such quantification scheme parameters are determined which are related to a microstructural property of the tissue. As described below in more detail, the present method explicitly uses a single statistical parameter for each of the selected quantification scheme parameters. Neither a fixed combination of static statistical parameters nor a repetitive combination of varying statistical parameters, both of which may be computed by mathematical variations of the quantification scheme parameters, is included in the method according to the present invention. As a result, further possible quantification scheme parameters which are only based on a mathematical manipulation of the original quantification scheme parameters, such as by forming a mean, a median, a range, a maximum, or a minimum value by using two or more already determined quantification scheme parameters, are not taken into account here. As a result, a reduced computation time may also be achieved, thus, contributing to prepare the present method for clinical use. According to the objective of the present invention, it may be, particularly, advantageous to keep the number of quantification scheme parameters within a reasonable limit, in particular between two and twenty, specifically between two and ten, in order to be able to achieve a comprehensive characterization of the different microstructural properties of the tissue during an evaluation time as short as possible in order to qualify the method as described herein for use in clinical routine. As a result, this procedure allows to closely approximate true microstructural tissue properties by application of DWI imaging since complex derived modifications, by using more than one statistical parameter, in particular a set of statistical parameters, derived from quantification scheme parameters as, for example, found in modern mathematical computational models, including but not limited to Skewness or Kurtosis, has proven not to closely reflect true tissue microstructural properties. Thus, as already mentioned above, by using the at least one quantification scheme, the at least two quantification scheme parameters are extracted from the raw MRI data, wherein the resulting quantification scheme parameters may be used in the subsequently described step c).

According to step c), a weight is applied to each quantification scheme parameter in a manner that a set of weighted quantification scheme parameters $\{k_i, p_i; i = 1 \dots n, n \geq 2\}$ is obtained. Herein, the set of weighted quantification scheme parameters comprises the at least two quantification scheme parameters, preferably two to twenty quantification scheme parameters, more preferred two to ten quantification scheme parameters, which have been extracted from the raw MRI data in accordance with step b), wherein each of the quantification scheme parameters $p_i$ has been furnished with a particular weight $k_i$, wherein each weight $k_i$ is dependent on a kind of the tissue under investigation and on the quantification scheme applied for determining the respective quantification scheme parameters $p_i$.

As generally used, the term "weight" refers to a factor which is applied in order to quantitatively express a particular property of each of a number of parameters which are comprised within a set. Herein, the weighting may, preferably, be adapted to at least one of: a tumor entity, a tumor subgroup, a particular quantification scheme, or a raw data training set, by which the weighted quantification of DWI sequences may be further improved. By way of example, the quantification scheme parameter $p_1$ may have a different weighting than the quantification scheme parameter $p_2$ although both may be determined from a single quantification scheme while, further, both weightings $k_1$, $k_2$ may differ between different kinds of tissues under investigation, e.g., they might be different for a breast tissue, a cervical tissue, and a prostate tissue. A number of examples regarding the quantification scheme parameters $p_i$ and the corresponding weights $k_i$ will be provided below. In order to provide a reliable set of weights, a training data set as described above may, preferably, be applied in order to determine the respective weights to be applied for a particular kind of the tissue and a selected quantification scheme.

In a preferred embodiment, at least one statistical approach can be used here for an assessment of the quantification schemes and the corresponding weightings. Herein, a specific quantification scheme may be processed for at least one of: a single voxel, a group of adjacent voxels, the lesion only, or the whole organ associated with the suspicious lesion. In particular, statistical parameters may be used which may be, especially, be selected from a mean, a median, a variance, or an entropy. However, other kinds of processes and/or other statistical approaches, such as those known to the persons familiar in the art, may also be applicable.

According to step d), a scoring value $Q$ is determined by combining the weighted quantification scheme parameters within the set of weighted quantification scheme parameters $\{k_i, p_i; i = 1 \dots n, n \geq 2\}$. As generally used, the term "scoring value" refers to a quantitative information which is applicable for classification purposes, wherein, as disclosed below in more detail, the quantity of the scoring value may, preferably, be compared with at least one score cut-off value in order to arrive at the class which corresponds to a particular scoring value. Herein, each of the extracted and, subsequently, weighted quantification scheme parameters is used only once for determining the scoring value. As a result, this feature implies that only a single statistical parameter, specifically a minimum, a maximum, a range, a mean, a median or a different statistical parameter, which may be related to or applied to a particular quantification scheme parameter, can, in particular, be included in determining the scoring value $Q$.

As a preferred example for determining the scoring value $Q$, the weighted quantification scheme parameters within the set $\{k_i, p_i; i = 1 \dots n, n \geq 2\}$ may be combined in accordance with Equation (3) as

$$Q = k_0 + \sum_{i=1}^{n \geq 2} k_i \cdot p_i, \qquad (3)$$

i.e. by summing up the $n$ selected weighted quantification scheme parameters $k_i \cdot p_i$. However, further schemes may also be applicable for reasonably combining the weighted quantification scheme parameters.

In a further preferred embodiment, a set of $m$ weighted additional data $\{\ell_j, q_j, j = 1 \dots m\}$ apart from the set of $n$ weighted quantification scheme parameters $\{k_i, p_i; i = 1 \dots n, n \geq 2\}$ as described above may be used to determine the scoring value. In this particular embodiment, the scoring value $Q$ may, preferably, be determined according to Equation (4) by

$$Q = k_0 + \sum_{i=1}^{n \geq 2} k_i \cdot p_i + \sum_{j=1}^{m} \ell_j \cdot q_j, \qquad (4)$$

which can be considered as a straight-forward modification of Equation (3). In particular, the additional data may be acquired by further modalities which may be used for tissue characterization, preferably by a non-invasive imaging modality, in particular, by ultrasound, x-ray imaging, computer tomography, positron emission tomography (PET), and/or conventional MRI, especially conventional MR sequencing. Alternatively or in addition, at least one further commonly used magnetic resonance imaging sequence may, additionally, be applied for sequencing. Alternatively or in addition, the additional data may comprise clinical data, in particular patient age, patient weight, patient origin, history of cancer in patient and/or family, a risk scoring model (such as a GAIL-model for breast cancer), an exposure to at least one risk factor potentially increasing the risk of having a malignancy (such as smoking, irradiation, exposure to chemical or biological substances), an infectious disease, a region of a lesion, at least one blood parameter, or a genetic analysis. However, other kinds of clinical data may also be applicable for this purpose.

According to step e), the tumorous modification of the tissue under investigation is classified into one of at least two classes according to the scoring value $Q$. Hereby, the tumorous tissue modification may be assigned to a particular class by comparing the scoring value $Q$ with at least one score cut-off value. As mentioned above, the term "score cut-off value" refers to a numerical value which is adapted for being used in discriminating between two adjacent classes. By way of example, the single score cut-off value may be set to "0", whereupon each positive scoring value $Q$ may be assigned into a first class while each negative scoring value $Q$ may be assigned into a second class being different from the first class. In a further example in which a discrimination between $s > 2$ classes may be employed, at least $s - 1$ score cut-off values may be applied for achieving the desired discrimination. However, other kinds of score cut-off values may also be feasible.

Although the method as described herein does not require an application of any contrast agent which may be administered to a patient comprising the tissue to be examined, still, relatively short examination and evaluation times can be achieved by concentrating the evaluation of the raw MRI data on those quantification scheme parameters which are actually related to microstructural properties of the tissue under examination. In particular, examination times of 0.5 min to 35 min, preferably of 5 min to 15 min, are preferred, wherein the term "examination time" refers to a period of time which is required for treating the tissue according to step a) with the at least one diffusion weighted imaging (DWI) sequence as applied by the MRI device. The further steps method b) to e) can, subsequently, be performed during a separate period of time which may be denoted by the term "evaluation time", during which a presence of the patient is, typically, not required. In particular due to the relatively short examination times as indicated above, the method as described herein appears to qualify for use in clinical routine.

In a further aspect, the present invention refers to a computer program product which comprises executable instructions for performing a method according to the present invention. For this purpose, a computer program may comprise instructions provided by means of a computer program code which are capable of performing any or all of the steps of the methods according to the present invention and, thus, to establish a classification process when implemented on a computer or a data processing device. The computer program code may be provided on a data storage medium or a separate device such as an optical storage medium, e.g., a compact disc, or directly on a computer or data processing device.

In a further aspect of the present invention, a classification device which is configured for non-invasively classifying a tumorous modification of a tissue into one of at least two classes, wherein each class refers to a different stage of the tumorous modification, is disclosed. Herein, the classification device comprises a receiving unit being adapted for performing at least step a) of the method according to the present invention and an evaluation unit being configured for performing at least steps b) to e) of the method according to the present invention.

Consequently, the receiving unit is adapted for receiving raw MRI data being recorded by applying at least one DWI sequence using three to nine different b-values to a tissue being suspicious to a tumorous modification without application of a contrast agent to the tissue, specifically to a body comprising the tissue. In a preferred embodiment as described below in more detail, the receiving unit may be arranged as a partition of a DWI parameter engine which, apart from, being adapted for receiving the raw MRI data, may further be configured for performing at least one further definite task within the evaluation unit of the classification device. In this particular embodiment, the raw MRI data may be provided by a magnetic resonance imaging device. However, other arrangements for the receiving unit may also be feasible.

In a particularly preferred embodiment, the evaluation unit may comprise a DWI parameter generator, a DWI parameter engine, and a scoring engine. Herein, the DWI parameter generator may be configured for providing at least one quantification scheme, preferably one to five quantification schemes, for further processing of the raw MRI data according to step b). Further, the DWI parameter engine may, in particular, be configured for extracting at least two quantification scheme parameters, preferably two to twenty quantification scheme parameters, from the raw MRI data as provided by the diffusion weighted imaging unit by using at least one quantification scheme as provided by the DWI parameter generator in accordance with step b). Further, the scoring engine may, especially be configured for providing a set of weighted quantification scheme parameters $\{k_i, p_i; i = 1 \dots n, n \geq 2\}$ according to step c), which may be obtained by applying a weight to each quantification scheme parameter as provided by the DWI parameter engine. Further, the scoring engine may, in particular, be configured for determining a scoring value by combining the weighted quantification scheme parameters $\{k_i, p_i; i = 1 \dots n, n \geq 2\}$ within the set in accordance with step d). Further, by using the scoring value, the scoring engine may, thus, also be configured for providing an assessment result being designated for classifying the tumorous modification of the tissue into one of at least two classes according to step e). Herein, the assessment result may be outputted to a decision output, wherein the decision output may comprise any form that may be suitable for providing and/or presenting the assessment result.

In a further preferred embodiment, the classification device may, further, comprise at least one optional component. Accordingly, the classification device may, further, comprise an adjacent context evaluation engine being adapted for providing a set of $m$ weighted additional data $\{\ell_j, q_j, j = 1 \dots m\}$ apart from the set of $n$ weighted quantification scheme parameters $\{k_i, p_i; i = 2 \dots n\}$ as provided by the DWI parameter engine on the basis of the raw MRI data to the scoring engine. As mentioned above, the additional data may be acquired by further modalities which may be used for tissue characterization, preferably, by a non-invasive imaging modality, in particular, by ultrasound, x-ray imaging, computer tomography, positron emission tomography (PET), and/or conventional MRI, especially conventional MR sequencing, especially for providing optional supplementary information to be included into the analysis of the tissue. Alternatively or in addition, the the classification device may, further, comprise a clinical information engine being configured for providing additional clinical data as described elsewhere herein in more detail.

In a further aspect of the present invention, a classification system is disclosed which comprises at least one classification device as already described herein and at least a magnetic resonance imaging (MRI) device. Herein, the MRI device is adapted for providing raw MRI data as described in step a). For this purpose, the MRI device may comprise at least one magnetic resonance unit being configured for performing a magnetic resonance examination of the tissue being suspicious to a tumorous modification. Further, the MRI device may comprise a sequencing unit being configured for providing MRI sequences in form of at least one DWI sequence to be used for the magnetic resonance examination of the tissue, preferably in a sequential manner. Further, the MRI device may comprise a diffusion weighted imaging unit being adapted for providing the raw MRI data as recorded by magnetic resonance imaging device to the classification device.

For further details concerning the classification device and the classification system, reference may, preferably, be made to the method according to the present invention as disclosed elsewhere in this document.

The method and the device according to the present invention provide considerable advantages over known methods and devices. In particular, the method according to the present invention is capable of providing a comprehensive weighted quantification of multiple microstructural tissue properties of different tissues using a single imaging sequence without intravenous contrast administration in a relatively short examination time independently from the magnetic resonance device used for providing the raw MRI data, wherein the examination is used in its entirety for classifying a potential tumorous modification of the tissue into one of at the least two classes. Non-invasive tissue characterization without contrast agent administration can, thus, be achieved in a highly accurate manner while supplementary information related to conventional imaging properties and to clinical information can further increase the high diagnostic accuracy. Although the present method and device could get rid of applying contrast agents, such as gadolinium-comprising compounds, to patients, still, a relatively short examination time and also a short evaluation time could, nevertheless, be achieved by concentrating the evaluation of the raw MRI data on quantification scheme parameters which are related to microstructural properties of the tissue under examination. As a result, this method and device appear, particularly, promising for use in clinical routine.

The method and the device according to the present invention, thus, provide opportunities for a combined weighted complementary quantification of DWI for microstructural correlates to produce a multi-parametric comprehensive characterization of the underlying tissue using one imaging sequence as a primary basis. A further combination with supplementary information may further support this approach. A illustrated below in more detail, a comparable or a higher diagnostic certainty could be achieved in contrast to current routine performance of using manual classification systems, such as BI-RADS (Breast Imaging Reading and Documentation System) [21] and PI-RADS (Prostate Imaging Reading and Documentation System) [22].

In further contrast to routine imaging, the method according to the present invention does not require long examination protocols, such as caused by either a large number of different image sequences or a large number of b-values in order to determine the quantification scheme parameters, contrast agent administration or invasive procedures. Contrast agents are known at risk for inducing allergic reactions up to severe complications, to potentially induce nephrogenic systemic fibrosis and gadolinium deposition in the human brain. These side effects need to be considered when administration of intravenous contrast agent is performed and the use of contrast agents is increasingly discussed and investigated such as by the FDA Drug Safety Communication of July 25, 2015. Another aspect of contrast agent administration is the high costs related to the administration hampering a broader use.

Long examination times in MRI may significantly increase the cost of the imaging method and shorten the availability to a broader patient audience, in particular, hinder an introduction of such methods and devices into clinical routine. In contrast hereto, the present method and device offer examination times of 0.5 min to 35 min, preferably of 5 min to 15 min, which considerably differ from examination times as known from experimental or scientific studies which, typically, exceed 30 min, 1 hour, or even more. Examination times can further be reduced since conventional imaging protocols commonly need a long reading time by the radiologist. The method according to the present invention does not only allow reducing peri-interventional complication rates but might also have a potential to significantly reduce unnecessary invasive biopsies, cost-effective re-examinations and potential harm not only in breast, cervix, and prostate malignancies which are considered as the most common tumors in females and males, respectively. Since invasive biopsies themselves are associated with further risks, a substantial clinical benefit is expected by using the present method invention.

Short description of the figures

Further optional details and features of the present invention may be derived from the subsequent description of preferred embodiments, preferably in combination with the dependent claims. Therein, the respective features may be realized in an isolated way or in arbitrary combinations. The invention is not restricted to the preferred embodiments. Identical reference numbers in the figures refer to identical elements or to elements having identical or similar functions or to elements corresponding to each other with regard to their functionality.

Figure 1 illustrates microstructural tissue correlates in benign and malignant breast lesions for three different solitary quantification schemes as extracted from diffusion weighted imaging (DWI) for comparison purposes (prior art);

Figure 2 illustrates microstructural tissue correlates in benign and malignant prostate lesions for three different solitary quantification schemes as extracted from DWI for comparison purposes (prior art);

Figure 3 illustrates the correlation between patient age and cancer risk for breast lesions for comparison purposes (prior art);

Figure 4 illustrates a classification system according to the present invention which comprises a magnetic resonance imaging device and a classification device;

Figure 5 illustrates microstructural tissue correlates in benign and malignant breast lesions as determined according to the present invention;

Figure 6 illustrates microstructural tissue correlates in clinically insignificant and clinically significant prostate lesions as determined according to the present invention; and

Figure 7 illustrates microstructural tissue correlates in clinically insignificant and clinically significant cervix lesions as determined according to the present invention.

Description of Preferred Embodiments

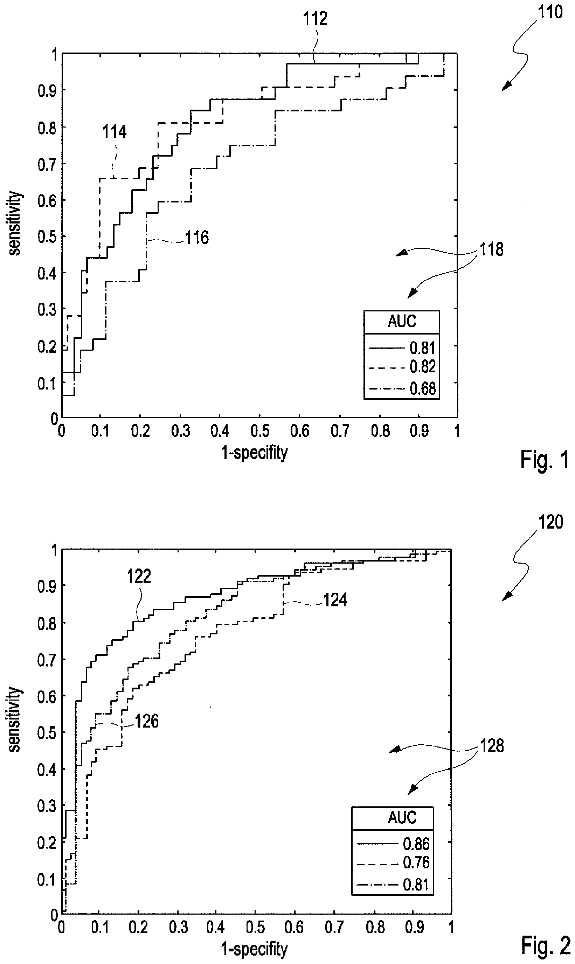

For comparison purposes, Figures 1 to 3 demonstrate that currently available solitary quantitative parameters based on diffusion weighted imaging (DWI) sequences for differentiating between benign and malignant tissue in magnetic resonance imaging (MRI) only exhibit of a rather limited diagnostic value.

In particular, Figure 1 illustrates a scheme 110 which comprises three different receiver operating curves (ROC) 112, 114, 116 for three different types of solitary quantification schemes, wherein each of the solitary quantification schemes was applied for determining microstructural tissue correlates in benign and malignant breast lesions as extracted from diffusion weighted imaging (DWI). Hereby, each of the curves 112, 114, 116 renders values of a sensitivity as depicted versus values of 1-specify, wherein each of the curves 112, 114, 116 represents one of the following different solitary quantification schemes, wherein

- the curve 112 refers to 'diffusional kurtosis imaging' (DKI)
- the curves 114, 116 refer to 'fractional order calculus' (FROC).

As generally used, the term "sensitivity" refers to a true positive rate, in particular, to a percentage of patients actually having a malignancy and who were correctly identified as having the malignancy. Further, the term "specificity" refers to a true negative rate, in particular, to a percentage of patients actually having a benign lesion and who were correctly identified as not having a malignancy. In addition, quantitative values for respective areas (AUC) 118 as determined under each of the curves 112, 114, 116 are indicated in the bottom right of Figure 1 and amount to values of 0.81, 0.82, and 0.68, respectively. Consequently, the correlation as derived from DWI in benign and malignant breast lesions by using solitary quantification schemes was found not to exceed the value of 82 %.

Similarly, Figure 2 illustrates a further scheme 120 comprising three different ROC curves 122, 124, 126, each representing a particular, different solitary quantification scheme, i.e. 'diffusional kurtosis imaging' (DKI), 'fractional order calculus' (FROC) and 'intravoxel incoherent motions' (IVIM)), wherein each of the solitary quantification schemes was applied for determining microstructural tissue correlates in benign and malignant prostate lesions extracted from DWI. Again, quantitative values for respective areas (AUC) 128 as determined under each of the ROC curves 122, 124, 126 are indicated in the bottom right of Figure 2 and amount to values of 0.86, 0.76, and 0.81, respectively. Consequently, the correlation as derived from DWI in benign and malignant prostate lesions by using solitary quantification schemes was found not to exceed the value of 86 %.

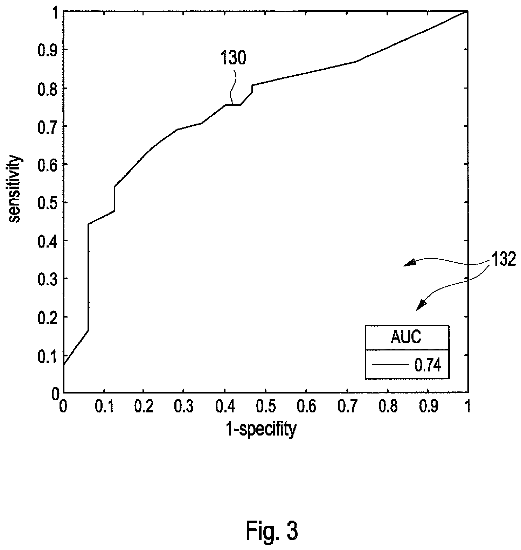

Further, Figure 3 illustrates a further ROC curve 130 with a further AUC area 132 under the further ROC curve 130, wherein patent age as a particular example for a clinical indicator may be employed as a potential addendum for the characterization of suspicious breast lesions. Again, a quantitative value for the AUC area 132 as determined under the further ROC curve 130 is indicated in the bottom right of Figure 3 and amounts to a value of 0.74. Consequently, taking only into account the patient age, the correlation between patient age and cancer risk for breast lesions was found already to assume the value of 74 %.

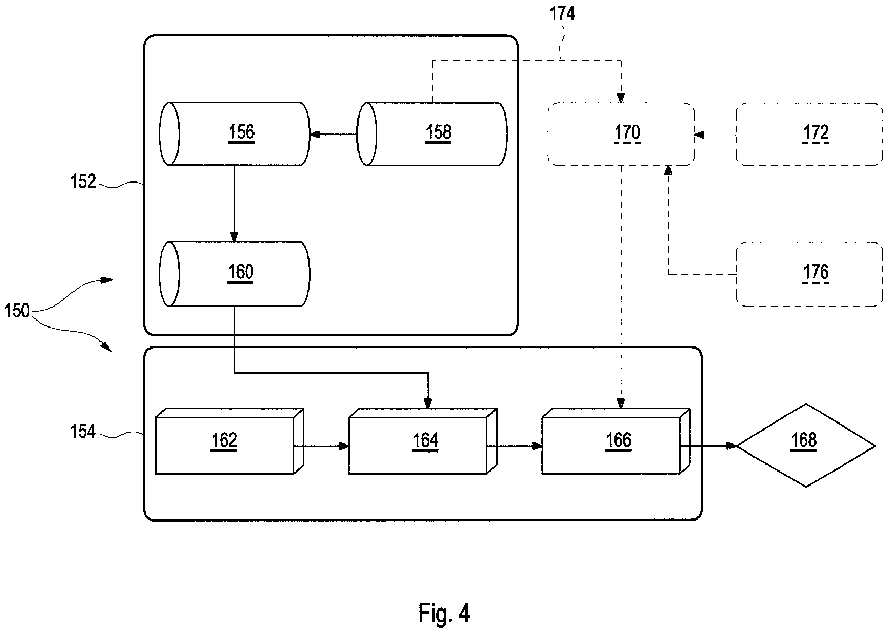

Figure 4 illustrates a classification system 150 according to the present invention which at least comprises a magnetic resonance imaging device 152 and a classification device 154.

As schematically depicted in Figure 4, the magnetic resonance imaging device 152 is adapted for providing raw magnetic resonance imaging (MRI) data as described in step a). For this purpose, the magnetic resonance imaging device 152 may comprise at least one magnetic resonance unit 156 which is configured for performing a magnetic resonance examination of a tissue being suspicious to a tumorous modification. Further, the magnetic resonance imaging device 152 may comprise a sequencing unit 158 which is configured for providing MRI sequences in form of at least one diffusion weighted imaging (DWI) sequence to be used for the magnetic resonance examination of the tissue, preferably in a sequential manner. Further, the magnetic resonance imaging device 152 may comprise a diffusion weighted imaging unit 160 which is adapted for providing the raw MRI data as recorded by magnetic resonance imaging device 152 by applying the at least one DWI sequence as generated by the sequencing unit 158 to the tissue being suspicious to the tumorous modification to the classification device 154.

The classification device 154 in the particularly preferred embodiment of Figure 4 comprises a DWI parameter generator 162, which is configured for providing at least one quantification schemes, preferably one to five different quantification schemes, more preferred two to four different quantification schemes, for further processing of the raw MRI data according to step b), wherein each of the quantification schemes and of quantification scheme parameters, preferably of two to twenty different quantification scheme parameters, more preferred of two to ten different quantification scheme parameters, as extracted from the raw MRI data by using the at least one selected quantification scheme, is related to a microstructural property of the tissue under investigation. For the purpose of receiving the raw MRI data as recorded by applying the at least one DWI sequence to the tissue according to step a), the classification device 154 further comprises a DWI parameter engine 164 which is, in addition, configured for extracting at least two quantification scheme parameters from the raw MRI data as provided by the diffusion weighted imaging unit 160 by using at least one quantification scheme as provided by the DWI parameter generator 162 in accordance with step b).

Further, the classification device 154 in the particularly preferred embodiment of Figure 4 comprises a scoring engine 166 which is configured for providing a set of weighted quantification scheme parameters $\{k_i, p_i; i = 1 ... n, n \geq 2\}$

according to step c), which is obtained by applying a weight to each quantification scheme parameter as provided by the DWI parameter engine 164, wherein the weight is dependent on a kind of the tissue and on the quantification scheme as provided by the DWI parameter generator 162. Further, the scoring engine 166 is configured for determining a scoring value by combining the weighted quantification scheme parameters $\{k_i, p_i; i = 1 ... n, n \geq 2\}$ within the set in accordance with step d). Further, the scoring engine 166 is configured for providing an assessment result by classifying the tumorous modification of the tissue into one of at least two classes according to the scoring value according to step e). As mentioned above, the tumorous modification, such as the modification of a breast tissue, may be sorted into one of the two classes benign or malignant. Alternatively, the tumorous modification, such as the modification of a prostate tissue, may be sorted into one of the three classes benign, clinically insignificant, or clinically significant. Further, the assessment result may be outputted to a decision output 168, wherein the decision output 168 may comprise any form that may be suitable for providing and/or presenting the assessment result.

In the embodiment as schematically depicted in Figure 4, the DWI parameter generator 162, the DWI parameter engine 164, and the scoring engine 166 may, jointly, be configured as an evaluation unit of the classification device 154 while, in this embodiment, the DWI parameter engine 164 may, additionally, be configured as a receiving unit being designated for receiving the raw MRI data provided by the resonance imaging device 152 to the classification device 154.

In the preferred embodiment of Figure 4, the classification device 154 may, further, comprise an adjacent context evaluation engine 170. Herein, the adjacent context evaluation engine 170 may be adapted for providing additional data $\{q_j, j = 1 ... m\}$ apart from the quantification scheme parameters $\{p_i; i = 1 ... n\}$ as provided by the DWI parameter engine 164 on the basis of the raw MRI data to the scoring engine 166. In this preferred embodiment, the scoring value Q may, preferably, be determined according to Equation (4) as presented above. In particular, the additional data may be acquired by further modalities which may be used for tissue characterization. For this purpose, the additional data may be provided to the adjacent context evaluation engine 170 by using a tissue morphology engine 172 which may be adapted for measuring and post-processing tissue-related data by a non-invasive imaging modality, in particular, by ultrasound, x-ray imaging, computer tomography, positron emission tomography (PET), and/or conventional MRI, especially conventional MR sequencing.

Alternatively or in addition, at least one further commonly used magnetic resonance imaging sequence 174 may, additionally, be applied for sequencing within the sequencing unit 158. In this embodiment, the further magnetic resonance imaging sequence 174 may, further, be used in the assessment by the scoring engine 166 to which it may be provided via the adjacent context evaluation engine 170 as optional supplementary information to be included into the analysis of the tissue.

Alternatively or in addition, the additional data may comprise clinical data, in particular patient age, patient weight, patient origin, history of cancer in patient and/or family, a risk scoring model (such as a GAIL-model for breast cancer), exposure to at least one risk factor potentially increasing the risk of having a malignancy (such as smoking, irradiation, exposure to chemical or biological substances), an infectious disease, a region of a lesion, at least one blood parameter, or a genetic analysis, which may be provided to the adjacent context evaluation engine 170 by using a clinical information engine 176 which may, particularly, be adapted for this purpose.

By using the adjacent context evaluation engine 170, the additional data can be provided to the scoring engine 166, where the additional data can be processed by combining them with the weighted quantification scheme parameters in order to determine the scoring value to be outputted to the decision output 168 as described above.

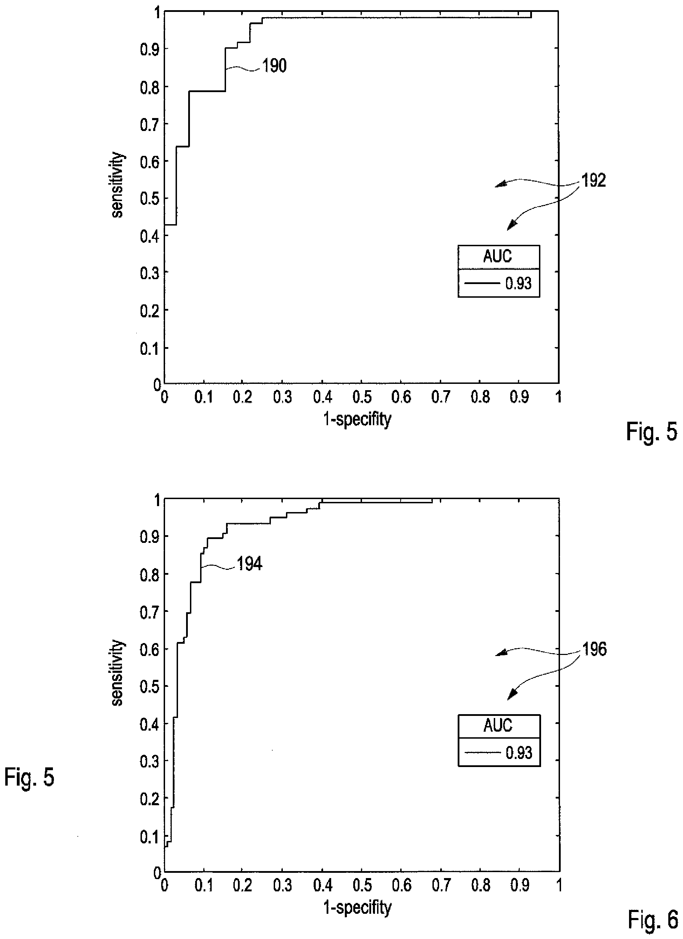

Figure 5 illustrates a ROC curve 190 and an AUC area 192 under the ROC curve 190, wherein the method and the device according the present invention was applied for a tissue suspicious of breast lesions. Again, a quantitative value for the AUC area 192 as determined under the further ROC curve 190 is indicated in the bottom right of Figure 5 and amounts to a value of 0.93. Consequently, by using the method and the device according the present invention, the AUC area 192 assumes the value of 93 % which is significantly higher compared to known procedures, such as illustrated in Figure 1.

Similarly, Figure 6 illustrates a ROC curve 194 and an AUC area 196 under the ROC curve 194, wherein the method and the device according the present invention was applied for a tissue suspicious of prostate lesions. Again, a quantitative value for the AUC area 196 as determined under the further ROC curve 194 is indicated in the bottom right of Figure 6 and amounts to a value of 0.93. Consequently, by using the method and the device according the present invention, the AUC area 196 assumes the value of 93 % which is, again, significantly higher compared to known procedures, such as illustrated in Figure 2.

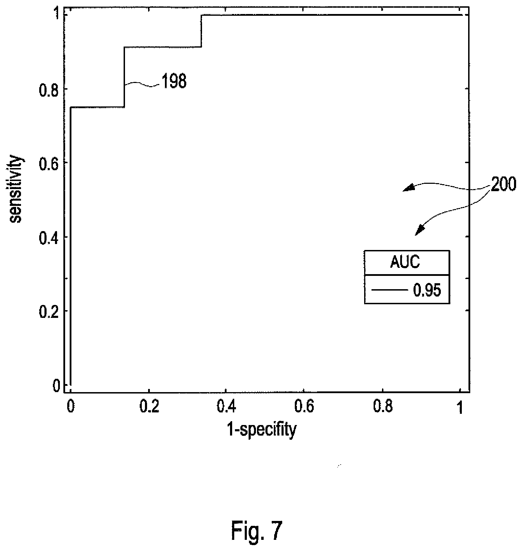

In addition, Figure 7 illustrates a ROC curve 198 and an AUC area 200 under the ROC curve 198, wherein the method and the device according the present invention was applied for a tissue suspicious of cervix lesions. Again, a quantitative value for the AUC area 200 as determined under the further ROC curve 198 is indicated in the bottom right of Figure 7 and amounts to a value of 0.95. Consequently, by using the method and the device according the present invention, the AUC area 200 assumes the value of 95 % which is, also, significantly higher compared to known procedures, such as illustrated in Figure 2.

Experimental results

Using the method and the device according to the present invention, a clear differentiation between malignant and benign tissue with regard to possible breast, cervix, and prostate cancer could be provided for approximately 400 patients.

**Table 1** provides an example of six patients with suspicious breast lesions which were classified by using the method according to the present invention, wherein each of 8 quantification scheme parameters $p_i$, i = 1 ... 8 were weighted with individual weights $k_i$, i = 1... 8, from which a scoring value $Q$ was determined according to Equation (3') as

$$Q = k_0 + \sum_{i=1}^{8} k_i \cdot p_i, \qquad (3')$$

i.e. by summing up the 8 selected weighted quantification scheme parameters $k_i \cdot p_i$. The scoring value $Q$ was used to classify the tissue modification into one of the two classes "benign" and "malignant" by applying a score cut-off value of 0.

**Table 1**

| Pat. | $p_1$ | $p_2$ | $p_3$ | $p_4$ | $p_5$ | $p_6$ | $p_7$ | $p_8$ | Q | Hist. | BI-RADS |
|---|---|---|---|---|---|---|---|---|---|---|---|
| 1 | 0.90 | 2.04 | 1.08 | 0.99 | 0.60 | 7.45 | 0.66 | 0.16 | -9,86 | malignant | 4 |
| 2 | 0.67 | 1.85 | 0.94 | 0.70 | 0.60 | 7.44 | 0.58 | 0,10 | -8,91 | malignant | 4 |
| 3 | 0.42 | 0.93 | 0.80 | 0.52 | 0.74 | 6.74 | 0.42 | 0.11 | -6,56 | malignant | 4 |
| 4 | 1.80 | 0.60 | 2.56 | 1.83 | 0.79 | 9.90 | 1.68 | 0.09 | 5,76 | benign | 4 |
| 5 | 2.24 | 0.68 | 2.97 | 2.91 | 0.82 | 14.80 | 2.02 | 0,20 | 5.34 | benign | 4 |
| 6 | 2.42 | 0.45 | 2.66 | 2.44 | 0.86 | 10.75 | 2.16 | 0.21 | 8.21 | benign | 4 |

In a similar manner, Table 2 provides an example of six patients with suspicious prostate lesions which were, again, classified by using the method according to the present invention, wherein each of 9 quantification scheme parameters $p_i$, i = 1 ... 9 were weighted with individual weights $k_i$, i = 1 ...9, from which a scoring value $Q$ was determined according to Equation (3") as

$$Q = k_0 + \sum_{i=1}^{9} k_i \cdot p_i, \qquad (3'')$$

i.e. by summing up the 9 selected weighted quantification scheme parameters $k_i \cdot p_i$. The scoring value $Q$ was, again, used to classify the tissue modification into one of the two classes "benign" and "malignant" by applying a score cut-off value of 0.

**Table 2**

| Pat. | $p_1$ | $p_2$ | $p_3$ | $p_4$ | $p_5$ | $p_6$ | $p_7$ | $p_8$ | $p_9$ | Q | Hist. | PI-RADS |
|---|---|---|---|---|---|---|---|---|---|---|---|---|
| 1 | 0.65 | 1.19 | 1.00 | 0.69 | 0.80 | 6.92 | 0.52 | 0.10 | 5.38 | -4,36 | malign. | 4 |
| 2 | 0.61 | 1.14 | 1.27 | 0.72 | 0.66 | 7.37 | 0.46 | 0.17 | 5.35 | -4,68 | malign. | 4 |
| 3 | 0.69 | 1.17 | 1.32 | 0.69 | 0.75 | 6.46 | 0.52 | 0.14 | 5.40 | -4,52 | malign. | 4 |
| 4 | 2.10 | 0.70 | 2.44 | 1.75 | 0.67 | 7.63 | 1.36 | 0.36 | 6.88 | 9,31 | benign | 4 |
| 5 | 2.07 | 0.81 | 2.29 | 1.91 | 0.55 | 8.55 | 1.21 | 0.39 | 6.82 | 9.22 | benign | 4 |
| 6 | 2.19 | 0.64 | 2.60 | 2.02 | 0.68 | 8.60 | 1.54 | 0.31 | 7.00 | 8.88 | benign | 4 |

In the following, three further examples are presented for providing further insight into the method according to the present invention.

**Example 1** refers to a patient for prostate cancer by check-up by MRI who was examined after a digital rectal examination with an unclear palpable finding. The patient underwent a prostate MRI examination in a 3T MRI device including a routine protocol as suggested by the PI-RADS ACR protocol. The protocol consisted of morphological sequences without contrast enhancement (T2-weighted), contrast enhanced sequences (T1-weighted) and a diffusion weighted imaging (DWI) sequence with multiple b-values according to the present invention.

After MRI examination the prostate was evaluated for a suspicious lesion as described in the PI-RADS V2 guidelines using the T2-weighted and DWI weighted imaging sequences. A lesion was detected and classified as a lesion coded with a PI-RADS class 3 ("intermediate"). The lesion was marked using a segmentation. The raw image data of the lesion was then processed using three different quantification schemes, i.e. Kurtosis, IVIM and the traditional monoexponential model, and three different quantification scheme parameters, i.e. $K_{Kurtosis}$, $f_{IVIM}$, and $D_{ADC}$, were extracted as described elsewhere in this document. The quantification scheme parameters were, subsequently, processed to finally receive a weighted scoring of each quantification scheme parameter resulting in a classifier to predict "clinically significant" or "clinically insignificant data". Herein, the weighting for each parameter was obtained of a raw data training set of approximately 200 patients with histopathologically confirmed lesions.

In particular, 3 quantification scheme parameters $p_1$ = 0.75, $p_2$ = 1.29, and $p_3$ = 0.15 were combined with 3 corresponding individual weights $k_1$ = 15.10, $k_2$ = 5.37, $k_3$ = -4.61 and $k_0$ = -25.09. Based on these data, the scoring value Q could be determined according to Equation (4) as Q = -25.09 + 15,10 • 0.75 + 5,37 • 1.29 + (-4,61) • 0.15 = -7,53. Applying a score cut-off value of 0, the suspicious prostate lesion was classified into the class "clinically significant", which corresponded with the result provided by the histopathology.

**Example 2** refers to a further patient for prostate cancer checkup by MRI who was examined after an elevated blood level of PSA had been found. The patient underwent a prostate MRI examination in the 3T MRI device including a routine protocol as suggested by the PI-RADS ACR protocol. The protocol consisted of morphological sequences without contrast enhancement (T2-weighted), contrast enhanced sequences (T1-weighted) and a diffusion weighted imaging (DWI) sequence with multiple b-values according to the present invention.

After MRI examination the prostate was evaluated for a suspicious lesion as described in the PI-RADS V2 guidelines using the T2-weighted and DWI weighted imaging sequences. A lesion was detected and classified as a lesion coded with a PI-RADS class 3 ("intermediate"). The lesion was marked using segmentation. The raw image data of the lesion was then processed using two different quantification schemes, i.e. Kurtosis, IVIM and the traditional monoexponential model, and three different quantification scheme parameters, i.e. $K_{Kurtosis}$, $f_{IVIM}$, and $D_{ADC}$, were extracted as described in the patent application. The quantification scheme parameters were, subsequently, processed to finally receive a weighted scoring of each quantification scheme parameter resulting in a classifier to predict "clinically significant" or "clinically insignificant data". Herein the weighting for each parameter was obtained of a raw data training set of approximately 200 patients with histopathologically confirmed lesions.

In particular, the 3 quantification scheme parameters $p_1$ = 2.06, $p_2$ = 0.62, and $p_3$ = 0.24 were, again, combined with the same 3 corresponding individual weights $k_1$ = 15.10, $k_2$ = 5.37, $k_3$ = - 4.61 and $k_0$ = -25.09 as in **Example 1** above. Based on these data, the scoring value Q could be determined according to Equation (4) as Q = -25.09 + 15,10 • 2.06 + 5,37 • 0.62 + (-4,61) • 0.24 = 8,24. Applying a score cut-off value of 0, the suspicious prostate lesion was classified into the class "clinically insignificant", which corresponded with the result provided by the histopathology.

**Example 3** refers to patient with a suspicious breast lesion who was examined due to a suspicious finding on X-ray mammography screening. The patient underwent a regular breast MRI scan using a 1.5 T MRI device. Herein, the acquired image sequences consisted of a regular breast imaging protocol with unenhanced T1-weighted and T2-weighted sequences, dynamic contrast enhanced T1-weighted imaging sequences, and a diffusion weighted imaging (DWI) sequence with multiple b-values according to the present invention.

A lesion was detected that provided an unclear rating according to the BI-RADS classification scheme (BI-RADS 3). Applying the invention procedure out of the detected and segmented lesion eight different quantification scheme parameters were extracted using different quantification schemes, i.e. the traditional monoexponential model, Kurtosis, IVIM, and FROC. With further allowing the scoring engine to not only use a weighted scoring of the quantification scheme parameters but also clinical information in terms of the patient age it was possible to classify this lesion as malignant which, being in contrast to the suggestion of the conventional BI-RADS criteria, was confirmed by histopathology. The weighting for the scoring engine was based on training of approximately 200 cases.

In particular, 8 quantification scheme parameters $p_1$ = 0.57, $p_2$ = 1.22, $p_3$ = 2.27, $p_4$ = 0.69, $p_5$ = 0.52, $p_6$ = 11.40, $p_7$ = 0.62, and $p_8$ = 0.38 were combined with 8 corresponding individual weights $k_1$ = 5.12, $k_2$ = -5.94, and $k_3$ = 0.8, $k_4$ = -3.38, $k_5$ = -9.95, $k_6$ = 0.23, $k_7$ = 4.55, $k_8$ = -10.69 and $k_0$ = 3.03. Based on these data, the scoring value Q could be determined according to Equation (4) as Q = 3,03 + 5,12 • 0.57 + (-5,94) • 1.22 + 0,80 • 2.27 + (-3,38) • 0.69 + (-9,95) • 0.52 + 0,23 • 11,40 + 4,55 • 0.62 + (-10,69) • 0.38 = -5,61. Applying a score cut-off value of 0, the suspicious prostate breast was classified into the class "malignant", which corresponded with the result provided by the histopathology.

As a result, the method according to the present invention allows determining different scoring values in malignant lesions compared to scoring values in benign lesions with a significant difference. The separation between malignant tissue and benign tissue for both prostate and breast lesions was found to be comparable or superior to conventional classification schemes of imaging using BI-RADS (Breast Imaging Reading and Documentation System) and PI-RADS (Prostate Imaging Reading and Documentation System) with conventional imaging protocols that include a combination of T1-weighted imaging before and after intravenous contrast administration, conventional DWI with ADC maps, and T2-weighted imaging.

Although limited to the description of using the method and device according to the present invention in breast and prostate tissue imaging, both the method and device are expected to be of added value for further kinds of tumorous modification in various tissues.

List of cited references

[1] Freitag MT, Bickelhaupt S, Ziener C, Meier-Hein K, Radtke JP, Mosebach J, et al. Selected clinically established and scientific techniques of diffusion-weighted MRI: In the context of imaging in oncology. Der Radiologe 2016;56(2):137-47.

[2] Radbruch A, Weberling LD, Kieslich PJ, Eidel O, Burth S, Kickingereder P, et al. Gadolinium retention in the dentate nucleus and globus pallidus is dependent on the class of contrast agent. Radiology. 2015;275(3):783-91.

[3] Errante Y, Cirimele V, Mallio CA, Di Lazzaro V, Zobel BB, Quattrocchi CC. Progressive increase of T1 signal intensity of the dentate nucleus on unenhanced magnetic resonance images is associated with cumulative doses of intravenously administered gadodiamide in patients with normal renal function, suggesting dechelation. Invest Radiol. 2014;49(10):685-90.

[4] Kanda T, Ishii K, Kawaguchi H, Kitajima K, Takenaka D. High signal intensity in the dentate nucleus and globus pallidus on unenhanced T1-weighted MR images: relationship with increasing cumulative dose of a gadolinium-based contrast material. Radiology. 2014;270(3):834-41.

[5] McDonald RJ, McDonald JS, Kallmes DF, Jentoft ME, Murray DL, Thielen KR, et al. Intracranial Gadolinium Deposition after Contrast-enhanced MR Imaging. Radiology. 2015;275(3):772-82.

[6] Hirano M et al. Diffusion-Weighted Imaging of Breast Masses: Comparison of Diagnostic Performance Using Various Apparent Diffusion Coefficient Parameters, Am. J. of Roentgenology 2012;198(3):717-722.

[7] Junker D et al. Evaluation of the PI-RADS Scoring System for Classifying mpMRI Findings in Men with Suspicion of Prostate Cancer, BioMed Research International 2013:1-9

[8] Othman E, Wang J, Sprague BL, Rounds T, Ji Y, Herschorn SD, et al. Comparison of false positive rates for screening breast magnetic resonance imaging (MRI) in high risk women performed on stacked versus alternating schedules. SpringerPlus. 2015;4:77.

[9] Baltzer PAT, Benndorf M, Dietzel M, Gajda M, Runnebaum IB, Kaiser WA. False-Positive Findings at Contrast-Enhanced Breast MRI: A BI-RADS Descriptor Study. American Journal of Roentgenology. 2010;194(6):1658-63.

[10] Quon JS, Moosavi B, Khanna M, Flood TA, Lim CS, Schieda N. False positive and false negative diagnoses of prostate cancer at multi-parametric prostate MRI in active surveillance. Insights into Imaging. 2015;6(4):449-63.

[11] Bammer R. Basic principles of diffusion-weighted imaging. Eur J Radiol. 2003; 45(3): 169-84.

[12] Koh DM, Collind DJ, Orton MR. Intravoxel incoherent motion in body diffusion-weighted MRI: reality and challenges. AJR Am J Roentgenol. 2011;196(6):1351-61.

[13] Yablonskiy DA, Bretthorst GL, Ackerman JJ. Statistical model for diffusion attenuated MR signal. Magnetic resonance in medicine. 2003;50(4):664-9.

[14] Jensen JH, Helpern JA, Ramani A, Lu H, Kaczynski K. Diffusional kurtosis imaging: the quantification of non-gaussian water diffusion by means of magnetic resonance imaging. Magnetic resonance in medicine. 2005;53(6):1432-40.

[15] Iima M, Yano K, Kataoka M, Umehana M, Murata K, Kanao S, et al. Quantitative non-Gaussian diffusion and intravoxel incoherent motion magnetic resonance imaging: differentiation of malignant and benign breast lesions. Invest Radiol. 2015;50(4):205-11.

[16] Sui Y, Wang H, Liu G, Damen FW, Wanamaker C, Li Y, et al. Differentiation of Low- and High-Grade Pediatric Brain Tumors with High b-Value Diffusion-weighted MR Imaging and a Fractional Order Calculus Model. Radiology. 2015;277(2):489-96.

[17] Panagiotaki E, Walker-Samuel S, Siow B, Johnson SP, Rajkumar V, Pedley RB, Lythgoe MF, and Alexander DC; Noninvasive Quantification of Solid Tumor Microstructure Using VERDICT MRI; Cancer Res; 74(7) April 1, 2014, 1902-12.

[18] Barbieri, S., Brönnimann, M., Boxler, S. et al. Differentiation of prostate cancer lesions with high and with low Gleason score by diffusion-weighted MRI. Eur Radiol 2016, pp 1-9.

[19] Sui, Y., H. Wang, et al. (2015). "Differentiation of Low- and High-Grade Pediatric Brain Tumors with High b-Value Diffusion-weighted MR Imaging and a Fractional Order Calculus Model." Radiology 277(2):489-496

[20] Montoya, P.I. et al. Diffusion weighted imaging of prostate cancer: Prediction of cancer using texture features from parametric maps of the monoexponential and kurtosis functions, 2016 Sixth International Conference on Image Processing Theory, Tools and Applications (IPTA), IEEE, 2016: 1-6

[21] Eberl MM, Fox CH, Edge SB, Carter CA, Mahoney MC. BI-RADS classification for management of abnormal mammograms. J Am Board Fam Med. 2006; 19(2):161-64.

[22] Weinreb JC, Barentsz JO, Choyke PL, Cornud F, Haider MA, Macura KJ, Margolis D, Schnall MD, Shtern F, Tempany CM, Thoeny HC, Verma S. Weinreb JC. PI-RADS Prostate Imaging-Reporting and Data System:2015,Version 2. Eur Urol 2016;69(1):16-40

List of reference numbers

[0088]

| 110 | scheme |
| 112 | ROC curve |
| 114 | ROC curve |
| 116 | ROC curve |
| 118 | AUC area |
| 120 | scheme |
| 122 | ROC curve |
| 124 | ROC curve |
| 126 | ROC curve |
| 128 | AUC area |
| 130 | ROC curve |
| 132 | AUC area |
| 150 | classification system |
| 152 | magnetic resonance imaging device |
| 154 | classification device |
| 156 | magnetic resonance unit |
| 158 | sequencing unit |
| 160 | diffusion weighted imaging unit |
| 162 | DWI parameter generator |
| 164 | DWI parameter engine |
| 166 | scoring engine |
| 168 | decision output |
| 170 | adjacent context evaluation engine |
| 172 | tissue morphology engine |
| 174 | magnetic resonance imaging sequence |

176    clinical information engine
190    ROC curve
192    AUC area
194    ROC curve
196    AUC area
198    ROC curve
200    AUC area

**Claims**

1. A computer-implemented method for non-invasively classifying a tumorous modification of a tissue into one of at least two classes, wherein each class refers to a different stage of the tumorous modification, wherein the method comprises the steps of:

   a) receiving raw magnetic resonance imaging (MRI) data that has been recorded by applying at least one diffusion weighted imaging (DWI) sequence using three to nine different b-values to a tissue being suspicious to a tumorous modification without application of a contrast agent;
   b) extracting at least two quantification scheme parameters from the raw MRI data by using two to four quantification schemes, wherein each quantification scheme is selected from 'diffusional kurtosis imaging' (DKI), "traditional monoexponential model', 'intravoxel incoherent motions' (IVIM), or 'fractional order calculus' (FROC), wherein a fitting procedure is applied for quantitatively determining the at least two quantification scheme parameters from the applied quantification schemes, wherein each of the quantification scheme parameters is related to a microstructural property of the tissue;
   c) applying a weight to each quantification scheme parameter, wherein the weight is dependent on a kind of the tissue and on the quantification scheme, whereby a set of weighted quantification scheme parameters is obtained;
   d) determining a scoring value by combining the weighted quantification scheme parameters within the set, wherein each of the weighted quantification scheme parameters is used only once for determining the scoring value such that only a single statistical parameter related to or applied to a particular quantification scheme parameter is included in determining the scoring value; and
   e) classifying the tumorous modification of the tissue into one of at least two classes according to the scoring value.

2. The method according to the preceding claim, wherein the tissue is a human tissue *in vivo* and wherein the tumorous modification is selected from the group consisting of breast cancer, cervix cancer, and prostate cancer.

3. The method according to any one of the preceding claims, wherein the stage of the tumorous modification is selected from one of

   - benign or malignant; or
   - benign, clinically insignificant, or clinically significant.

4. The method according to any one of the preceding claims, wherein the b-value is correlated with a magnetic field gradient as used for generating the DWI sequence.

5. The method according to the preceding claim, wherein the b-value is selected from a range of 0 to 4000 s/mm$^2$, wherein two adjacent b-values are separated from each other by at least 50 s/ mm$^2$.

6. The method according to any one of the preceding claims, wherein the quantification scheme parameter is selected from ADC; AKC; D-IVIM, or F-IVIM.

7. The method according to any one of the preceding claims, wherein the weight to each quantification scheme parameter is obtained by analyzing at least one training data set, wherein the training data set refers to data comprising a confirmed histopathological analysis.

8. The method according to any one of the preceding claims, wherein the scoring value $Q$ is determined by combining the weighted quantification scheme parameters within the set $\{k_i, p_i; i = 1 \ldots n, n \geq 2\}$ in accordance with Equation (3) as

$$Q = k_0 + \sum_{i=1}^{n \geq 2} k_i \cdot p_i, \qquad (3)$$

9. The method according to the preceding claim, wherein a set of $m$ weighted additional data $\{\ell_j, q_j, j = 1 \ldots m\}$ is, additionally, used for determining the scoring value in accordance with Equation (4) by

$$Q = k_0 + \sum_{i=1}^{n \geq 2} k_i \cdot p_i + \sum_{j=1}^{m} \ell_j \cdot q_j, \qquad (4)$$

10. The method according to the preceding claim, wherein additional data has been obtained from at least one of: a non-invasive imaging modality and clinical data.

11. The method according to the preceding claim, wherein a non-invasive imaging modality comprises at least one of: ultrasound, x-ray imaging, computer tomography, positron emission tomography (PET), or conventional MR sequencing, and wherein the clinical data comprises at least one of: patient age, patient weight, patient origin, history of cancer in patient and/or family, a risk scoring model, an exposure to at least one risk factors potentially increasing the risk of having a malignancy, an infectious disease, a region of a lesion, at least one blood parameter, or a genetic analysis.

12. The method according to any one of the preceding claims, wherein the scoring value is compared with at least one score cut-off value, by which the tumorous modification of the tissue is classified into one of the at least two classes.

13. A computer program product comprising executable instructions for performing a method according to any one of the preceding claims.

14. A classification device (154) for non-invasively classifying a tumorous modification of a tissue into one of at least two classes, wherein each class refers to a different stage of the tumorous modification, comprising

- a receiving unit for receiving raw magnetic resonance imaging (MRI) data being recorded by applying at least one diffusion weighted imaging (DWI) sequence using three to nine different b-values to a tissue being suspicious to a tumorous modification without application of a contrast agent; and
- an evaluation unit comprising a DWI parameter generator (162), a DWI parameter engine (164), and a scoring engine (166), wherein, the DWI parameter generator (162) is configured for providing two to four quantification schemes for further processing of the raw MRI data, wherein the DWI parameter engine (164) is configured for extracting at least two quantification scheme parameters from the raw MRI data by using the two to four quantification schemes, wherein the quantification scheme is selected from 'diffusional kurtosis imaging' (DKI), "traditional monoexponential model', 'intravoxel incoherent motions' (IVIM), or 'fractional order calculus' (FROC), wherein a fitting procedure is applied for quantitatively determining the at least two quantification scheme parameters from the applied quantification scheme, wherein each of the quantification scheme parameters is related to a microstructural property of the tissue, and wherein the scoring engine (166) is configured for providing a set of weighted quantification scheme parameters, for determining a scoring value by combining the weighted quantification scheme parameters, wherein each of the weighted quantification scheme parameters is used only once for determining the scoring value such that only a single statistical parameter related to or applied to a particular quantification scheme parameter is included in determining the scoring value, and, by using the scoring value, for classifying the tumorous modification of the tissue into one of at least two classes.

15. The classification device (154) according to the preceding claim, further comprising a adjacent context evaluation engine (170) being adapted for providing additional data, wherein the additional data is obtained from at least one of: a tissue morphology engine (172) and clinical information engine (176).

**Patentansprüche**

1. Computerimplementiertes Verfahren zum nicht invasiven Klassifizieren einer tumorartigen Modifikation eines Gewebes in eine von mindestens zwei Klassen, wobei sich jede Klasse auf ein anderes Stadium der tumorartigen Modifikation bezieht, wobei das Verfahren die folgenden Schritte umfasst:

a) Empfangen von Rohdaten der Magnetresonanztomografie (MRI), die durch Anwenden mindestens einer diffusionsgewichteten Bildgebungssequenz (DWI) unter Verwendung von drei bis neun verschiedenen b-Werten auf ein Gewebe, das einer tumorartigen Modifikation verdächtig ist, ohne Anwendung eines Kontrastmittels aufgezeichnet worden sind;

b) Extrahieren von mindestens zwei Quantifizierungsschema-Parametern aus den MRI-Rohdaten unter Verwendung von zwei bis vier Quantifizierungsschemata, wobei jedes Quantifizierungsschema ausgewählt ist aus ‚diffusional kurtosis imaging' (DKI), ‚traditional monoexponential model', ‚intravoxel incoherent motions' (IVIM), oder, fractional order calculus' (FROC), wobei ein Anpassungsvorgang angewandt wird, um die mindestens zwei Quantifizierungsschema-Parameter aus den angewandten Quantifizierungsschemata quantitativ zu bestimmen, wobei jeder Quantifizierungsschema-Parameter mit einer mikrostrukturellen Eigenschaft des Gewebes verbunden ist;

c) Anwenden einer Gewichtung auf jeden Quantifizierungsschema-Parameter, wobei die Gewichtung von einer Art des Gewebes und von dem Quantifizierungsschema abhängt, wodurch ein Satz gewichteter Quantifizierungsschema-Parameter erhalten wird;

d) Bestimmen eines Scoring-Wertes durch Kombinieren der gewichteten Quantifizierungsschema-Parameter innerhalb des Satzes, wobei jeder der gewichteten Quantifizierungsschema-Parameter nur einmal zum Bestimmen des Scoring-Wertes verwendet wird, so dass nur ein einziger statistischer Parameter, der sich auf einen bestimmten Quantifizierungsschema-Parameter bezieht oder auf diesen angewendet wird, beim Bestimmen des Scoring-Wertes beinhaltet ist; und

e) Klassifizieren der tumorartigen Modifikation des Gewebes in eine von mindestens zwei Klassen gemäß dem Scoring-Wert.

2. Verfahren nach dem vorhergehenden Anspruch, wobei das Gewebe ein menschliches Gewebe *in vivo* ist und wobei die tumorartige Modifikation aus der Gruppe bestehend aus Brustkrebs, Gebärmutterhalskrebs und Prostatakrebs ausgewählt ist.

3. Verfahren nach einem der vorhergehenden Ansprüche, wobei das Stadium der tumorartigen Modifikation ausgewählt ist aus einem der folgenden

- gutartig oder bösartig; oder
- gutartig, klinisch unbedeutend oder klinisch bedeutsam.

4. Verfahren nach einem der vorhergehenden Ansprüche, wobei der b-Wert mit einem Magnetfeldgradienten korreliert ist, wie er beim Erzeugen der DWI-Sequenz verwendet wird.

5. Verfahren nach dem vorhergehenden Anspruch, wobei der b-Wert aus einem Bereich von 0 bis 4000 s/mm$^2$ ausgewählt wird, wobei zwei benachbarte b-Werte durch mindestens 50 s/mm$^2$ voneinander getrennt sind.

6. Verfahren nach einem der vorhergehenden Ansprüche, wobei der Quantifizierungsschema-Parameter ausgewählt ist aus ADC; AKC; D-IVIM oder f-IVIM.

7. Verfahren nach einem der vorhergehenden Ansprüche, wobei die Gewichtung für jeden Quantifizierungsschema-Parameter durch Analysieren mindestens eines Trainingsdatensatzes erhalten wird, wobei sich der Trainingsdatensatz auf Daten bezieht, die eine bestätigte histopathologische Analyse umfassen.

8. Verfahren nach einem der vorhergehenden Ansprüche, wobei der Scoring-Wert $Q$ durch Kombinieren der gewichteten Quantifizierungsschema-Parameter innerhalb des Satzes $\{k_i, p_i; i = 1 \dots n, n \geq 2\}$ in Übereinstimmung mit Gleichung (3) bestimmt wird als

$$Q = k_0 + \sum_{i=1}^{n \geq 2} k_i \cdot p_i$$

9. Verfahren nach dem vorhergehenden Anspruch, wobei ein Satz von $m$ gewichteten zusätzlichen Daten $\{\ell_j, q_j, j = 1 \dots m\}$ zusätzlich verwendet wird, um den Scoring-Wert gemäß Gleichung (4) zu bestimmen, durch

$$Q = k_0 + \sum_{i=1}^{n \geq 2} k_i \cdot p_i + \sum_{j=1}^{m} \ell_j \cdot q_j$$

**10.** Verfahren nach dem vorhergehenden Anspruch, wobei zusätzliche Daten aus einer nicht invasiven Bildgebungsmodalität und/oder klinischen Daten erhalten werden.

**11.** Verfahren nach dem vorhergehenden Anspruch, wobei eine nicht invasive Bildgebungsmodalität umfasst: Ultraschall und/oder Röntgenbildgebung und/oder Computertomografie und/oder Positronen-Emissions-Tomografie (PET) und/oder konventionelle MR-Sequenzierung, und wobei die klinischen Daten umfassen: Alter des Patienten und/oder Gewicht des Patienten und/oder Herkunft des Patienten und/oder Verlauf der Krebserkrankung beim Patienten und/oder in der Familie und/oder ein Risiko-Scoring-Modell und/oder eine Exposition gegenüber mindestens einem Risikofaktor, der das Risiko für eine bösartige Erkrankung erhöhen könnte und/oder eine Infektionskrankheit und/oder eine Region einer Läsion und/oder mindestens einen Blutparameter und/oder eine genetische Analyse.

**12.** Verfahren nach einem der vorhergehenden Ansprüche, wobei der Scoring-Wert mit mindestens einem Score-Cut-off-Wert verglichen wird, durch den die tumorartige Modifikation des Gewebes in eine der mindestens zwei Klassen klassifiziert wird.

**13.** Computerprogrammprodukt, das ausführbare Anweisungen zum Durchführen eines Verfahrens nach einem der vorhergehenden Ansprüche umfasst.

**14.** Klassifizierungsvorrichtung (154) zum nicht invasiven Klassifizieren einer tumorartigen Modifikation eines Gewebes in eine von mindestens zwei Klassen, wobei sich jede Klasse auf ein anderes Stadium der tumorartigen Modifikation bezieht, umfassend

- eine Empfangseinheit zum Empfangen von Rohdaten der Magnetresonanztomografie (MRI), die durch Anwenden mindestens einer diffusionsgewichteten Bildgebungssequenz (DWI) unter Verwendung von drei bis neun verschiedenen b-Werten auf ein Gewebe, das einer tumorartigen Modifikation verdächtig ist, ohne Anwendung eines Kontrastmittels aufgezeichnet werden; und
- eine Auswerteeinheit, umfassend einen DWI-Parameter-Generator (162), eine DWI-Parameter-Engine (164) und eine Scoring-Engine (166), wobei der DWI-Parameter-Generator (162) zum Bereitstellen von zwei bis vier Quantifizierungsschemata zur weiteren Verarbeitung der MRI-Rohdaten ausgelegt ist, wobei die DWI-Parameter-Engine (164) zum Extrahieren von mindestens zwei Quantifizierungsschema-Parametern aus den MRI-Rohdaten unter Verwendung der zwei bis vier Quantifizierungsschemata ausgelegt ist, wobei das Quantifizierungsschema ausgewählt ist aus ,diffusional kurtosis imaging' (DKI), ,traditional monoexponential model', ,intravoxel incoherent motions' IVIM oder, fractional order calculus' (FROC), wobei ein Anpassungsvorgang angewendet wird, um die mindestens zwei Quantifizierungsschema-Parameter aus dem angewandten Quantifizierungsschema quantitativ zu bestimmen, wobei jeder der Quantifizierungsschema-Parameter mit einer mikrostrukturellen Eigenschaft des Gewebes in Beziehung steht, und wobei die Scoring-Engine (166) zum Bereitstellen eines Satzes gewichteter Quantifizierungsschema-Parameter, zum Bestimmen eines Scoring-Wertes durch Kombinieren der gewichteten Quantifizierungsschema-Parameter ausgelegt ist, wobei jeder der gewichteten Quantifizierungsschema-Parameter nur einmal zum Bestimmen des Scoring-Wertes verwendet wird, so dass nur ein einziger statistischer Parameter, der sich auf einen bestimmten Quantifizierungsschema-Parameter bezieht oder auf diesen angewendet wird, beim Bestimmen des Scoring-Wertes beinhaltet ist, und, unter Verwendung des Scoring-Wertes, zum Klassifizieren der tumorartigen Modifikation des Gewebes in eine von mindestens zwei Klassen.

**15.** Klassifizierungsvorrichtung (154) nach dem vorhergehenden Anspruch, die ferner eine benachbarte Kontextevaluierungs-Engine (170) umfasst, die zum Bereitstellen zusätzlicher Daten angepasst ist, wobei die zusätzlichen Daten erhalten werden von: einer Gewebemorphologie-Engine (172) und/oder einer klinischen Informations-Engine (176).

## Revendications

**1.** Procédé, mis en œuvre par ordinateur, de classification non invasive d'une modification tumorale d'un tissu en l'une d'au moins deux classes, chaque classe se rapportant à un stade différent de la modification tumorale, le procédé comprenant les étapes suivantes :

a) réception de données brutes d'imagerie par résonance magnétique (IRM) qui ont été enregistrées par application d'au moins une séquence d'imagerie pondérée en diffusion (DWI) à l'aide de trois à neuf valeurs de b différentes à un tissu faisant suspecter une modification tumorale sans application d'un agent de contraste ;

b) extraction d'au moins deux paramètres de schéma de quantification à partir des données brutes d'IRM au moyen de deux à quatre schémas de quantification, chaque schéma de quantification étant sélectionné parmi les schémas suivants : 'imagerie par kurtosis de diffusion' (DKI), 'modèle mono-exponentiel classique', 'mouvements incohérents intravoxéliens' (IVIM), ou 'calcul d'ordre fractionnaire' (FROC), une procédure d'ajustement étant appliquée pour la détermination quantitative des au moins deux paramètres de schéma de quantification à partir des schémas de quantification appliqués, chacun des paramètres de schéma de quantification étant lié à une propriété microstructurale du tissu ;

c) application d'un poids à chaque paramètre de schéma de quantification, le poids étant fonction d'un type du tissu et du schéma de quantification, un ensemble de paramètres de quantification pondérés étant ainsi obtenu ;

d) détermination d'une valeur de score par combinaison des paramètres de schéma de quantification pondérés au sein de l'ensemble, chacun des paramètres de schéma de quantification pondérés étant utilisé une seule fois pour la détermination de la valeur de score de telle sorte qu'un seul et unique paramètre statistique lié ou appliqué à un paramètre de schéma de quantification particulier soit pris en compte dans la détermination de la valeur de score ; et

e) classification de la modification tumorale du tissu en l'une d'au moins deux classes selon la valeur de score.

2. Procédé selon la revendication précédente, dans lequel le tissu est un tissu humain *in vivo,* et dans lequel la modification tumorale est sélectionnée dans le groupe comprenant un cancer du sein, un cancer du col de l'utérus, et un cancer de la prostate.

3. Procédé selon l'une quelconque des revendications précédentes, dans lequel le stade de la modification tumorale est sélectionné parmi soit :

   - bénin ou malin ; soit
   - bénin, cliniquement non significatif, ou cliniquement significatif.

4. Procédé selon l'une quelconque des revendications précédentes, dans lequel la valeur de b est corrélée avec un gradient de champ magnétique utilisé pour la génération de la séquence DWI.

5. Procédé selon la revendication précédente, dans lequel la valeur de b est sélectionnée dans une plage allant de 0 à 4000 s/mm$^2$, deux valeurs de b adjacentes étant séparées l'une de l'autre d'au moins 50 s/mm$^2$.

6. Procédé selon l'une quelconque des revendications précédentes, dans lequel le paramètre de schéma de quantification est sélectionné parmi ADC ; AKC ; D-IVIM, ou f-IVIM.

7. Procédé selon l'une quelconque des revendications précédentes, dans lequel le poids appliqué à chaque paramètre de schéma de quantification est obtenu par analyse d'au moins un ensemble de données d'entraînement, l'ensemble de données d'entraînement se rapportant à des données comprenant une analyse histopathologique confirmée.

8. Procédé selon l'une quelconque des revendications précédentes, dans lequel la valeur de score $Q$ est déterminée par combinaison des paramètres de schéma de quantification pondérés au sein de l'ensemble $\{k_i, p_i ; i = 1...n, n \geq 2\}$ conformément à l'Équation (3) suivante

$$Q = k_0 + \sum_{i=1}^{n \geq 2} k_i \cdot p_i, \qquad (3)$$

9. Procédé selon la revendication précédente, dans lequel un ensemble de $m$ données supplémentaires pondérées $\{\ell_j, q_j, j = 1...m\}$ est, de plus, utilisé pour la détermination de la valeur de score conformément à l'Équation (4) par

$$Q = k_0 + \sum_{i=1}^{n \geq 2} k_i \cdot p_i + \sum_{j=1}^{m} \ell_j \cdot q_j, \qquad (4)$$

10. Procédé selon la revendication précédente, dans lequel des données supplémentaires ont été obtenues à partir d'une modalité d'imagerie non invasive et/ou de données cliniques.

11. Procédé selon la revendication précédente, dans lequel une modalité d'imagerie non invasive comprend au moins une des modalités suivantes : les ultrasons, l'imagerie par rayons X, la tomographie assistée par ordinateur, la tomographie par émission de positrons (PET), ou le séquençage par résonance magnétique classique, et dans lequel les données cliniques comprennent au moins un des éléments suivants : l'âge du patient, le poids du patient, l'origine du patient, un antécédent de cancer chez le patient et/ou familial, un modèle de score de risque, une exposition à au moins un facteur de risque augmentant potentiellement le risque d'être atteint d'une malignité, une maladie infectieuse, une région d'une lésion, au moins un paramètre sanguin, ou une analyse génétique.

12. Procédé selon l'une quelconque des revendications précédentes, dans lequel la valeur de score est comparée à au moins une valeur seuil de score pour ainsi permettre la classification de la modification tumorale du tissu en l'une des au moins deux classes.

13. Produit-programme d'ordinateur comprenant des instructions exécutables pour réaliser un procédé selon l'une quelconque des revendications précédentes.

14. Dispositif de classification (154) pour la classification non invasive d'une modification tumorale d'un tissu en l'une d'au moins deux classes, chaque classe se rapportant à un stade différent de la modification tumorale, comprenant

   - une unité de réception pour recevoir des données brutes d'imagerie par résonance magnétique (IRM) qui sont enregistrées par application d'au moins une séquence d'imagerie pondérée en diffusion (DWI) à l'aide de trois à neuf valeurs de b différentes à un tissu faisant suspecter une modification tumorale sans application d'un agent de contraste ; et
   - une unité d'évaluation comprenant un générateur de paramètres DWI (162), un moteur de paramètres DWI (164), et un moteur de score (166), le générateur de paramètres DWI (162) étant configuré pour fournir de deux à quatre schémas de quantification pour un traitement plus poussé des données brutes d'IRM, le moteur de paramètres DWI (164) étant configuré pour extraire au moins deux paramètres de schéma de quantification à partir des données brutes d'IRM au moyen des deux à quatre schémas de quantification, le schéma de quantification étant sélectionné parmi les schémas suivants : 'imagerie par kurtosis de diffusion' (DKI), 'modèle mono-exponentiel classique', 'mouvements incohérents intravoxéliens' (IVIM), ou 'calcul d'ordre fractionnaire' (FROC), une procédure d'ajustement étant appliquée pour la détermination quantitative des au moins deux paramètres de schéma de quantification à partir du schéma de quantification appliqué, chacun des paramètres de schéma de quantification étant lié à une propriété microstructurale du tissu, et le moteur de score (166) étant configuré pour fournir un ensemble de paramètres de schéma de quantification pondérés, pour déterminer une valeur de score par combinaison des paramètres de schéma de quantification pondérés, chacun des paramètres de schéma de quantification pondérés étant utilisé une seule fois pour la détermination de la valeur de score de telle sorte qu'un seul et unique paramètre statistique lié ou appliqué à un paramètre de schéma de quantification particulier soit pris en compte dans la détermination de la valeur de score, et, au moyen de la valeur de score, pour classifier la modification tumorale du tissu en l'une d'au moins deux classes.

15. Dispositif de classification (154) selon la revendication précédente, comprenant en outre un moteur d'évaluation de contexte adjacent (170) adapté à fournir des données supplémentaires, les données supplémentaires étant obtenues à partir d'un moteur de morphologie tissulaire (172) et/ou d'un moteur d'informations cliniques (176).

Fig. 1

Fig. 2

Fig. 3

Fig. 4

EP 3 585 248 B1

Fig. 5

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FREITAG MT ; BICKELHAUPT S ; ZIENER C ; MEIER-HEIN K ; RADTKE JP ; MOSEBACH J et al.** Selected clinically established and scientific techniques of diffusion-weighted MRI: In the context of imaging in oncology. *Der Radiologe,* 2016, vol. 56 (2), 137-47
- **RADBRUCH A ; WEBERLING LD ; KIESLICH PJ ; EIDEL O ; BURTH S ; KICKINGEREDER P et al.** Gadolinium retention in the dentate nucleus and globus pallidus is dependent on the class of contrast agent. *Radiology,* 2015, vol. 275 (3), 783-91
- **ERRANTE Y ; CIRIMELE V ; MALLIO CA ; DI LAZZARO V ; ZOBEL BB ; QUATTROCCHI CC.** Progressive increase of T1 signal intensity of the dentate nucleus on unenhanced magnetic resonance images is associated with cumulative doses of intravenously administered gadodiamide in patients with normal renal function, suggesting dechelation. *Invest Radiol.,* 2014, vol. 49 (10), 685-90
- **KANDA T ; ISHII K ; KAWAGUCHI H ; KITAJIMA K ; TAKENAKA D.** High signal intensity in the dentate nucleus and globus pallidus on unenhanced T1-weighted MR images: relationship with increasing cumulative dose of a gadolinium-based contrast material. *Radiology,* 2014, vol. 270 (3), 834-41
- **MCDONALD RJ ; MCDONALD JS ; KALLMES DF ; JENTOFT ME ; MURRAY DL ; THIELEN KR et al.** Intracranial Gadolinium Deposition after Contrast-enhanced MR Imaging. *Radiology,* 2015, vol. 275 (3), 772-82
- **HIRANO M et al.** Diffusion-Weighted Imaging of Breast Masses: Comparison of Diagnostic Performance Using Various Apparent Diffusion Coefficient Parameters. *Am. J. of Roentgenology,* 2012, vol. 198 (3), 717-722
- **JUNKER D et al.** Evaluation of the PI-RADS Scoring System for Classifying mpMRI Findings in Men with Suspicion of Prostate Cancer. *BioMed Research International,* 2013, 1-9
- **OTHMAN E ; WANG J ; SPRAGUE BL ; ROUNDS T ; JI Y ; HERSCHORN SD et al.** Comparison of false positive rates for screening breast magnetic resonance imaging (MRI) in high risk women performed on stacked versus alternating schedules. *SpringerPlus,* 2015, vol. 4, 77

- **BALTZER PAT ; BENNDORF M ; DIETZEL M ; GAJDA M ; RUNNEBAUM IB ; KAISER WA.** False-Positive Findings at Contrast-Enhanced Breast MRI: A BI-RADS Descriptor Study. *American Journal of Roentgenology,* 2010, vol. 194 (6), 1658-63
- **QUON JS ; MOOSAVI B ; KHANNA M ; FLOOD TA ; LIM CS ; SCHIEDA N.** False positive and false negative diagnoses of prostate cancer at multi-parametric prostate MRI in active surveillance. *Insights into Imaging.,* 2015, vol. 6 (4), 449-63
- **BAMMER R.** Basic principles of diffusion-weighted imaging. *Eur J Radiol.,* 2003, vol. 45 (3), 169-84
- **KOH DM ; COLLIND DJ ; ORTON MR.** Intravoxel incoherent motion in body diffusion-weighted MRI: reality and challenges. *AJR Am J Roentgenol.,* 2011, vol. 196 (6), 1351-61
- **YABLONSKIY DA ; BRETTHORST GL ; ACKERMAN JJ.** Statistical model for diffusion attenuated MR signal. *Magnetic resonance in medicine,* 2003, vol. 50 (4), 664-9
- **JENSEN JH ; HELPERN JA ; RAMANI A ; LU H ; KACZYNSKI K.** Diffusional kurtosis imaging: the quantification of non-gaussian water diffusion by means of magnetic resonance imaging. *Magnetic resonance in medicine,* 2005, vol. 53 (6), 1432-40
- **LIMA M ; YANO K ; KATAOKA M ; UMEHANA M ; MURATA K ; KANAO S et al.** Quantitative non-Gaussian diffusion and intravoxel incoherent motion magnetic resonance imaging: differentiation of malignant and benign breast lesions. *Invest Radiol.,* 2015, vol. 50 (4), 205-11
- **SUI Y ; WANG H ; LIU G ; DAMEN FW ; WANAMAKER C ; LI Y et al.** Differentiation of Low- and High-Grade Pediatric Brain Tumors with High b-Value Diffusion-weighted MR Imaging and a Fractional Order Calculus Model. *Radiology.,* 2015, vol. 277 (2), 489-96
- **PANAGIOTAKI E ; WALKER-SAMUEL S ; SIOW B ; JOHNSON SP ; RAJKUMAR V ; PEDLEY RB ; LYTHGOE MF ; ALEXANDER DC.** Noninvasive Quantification of Solid Tumor Microstructure Using VERDICT MRI. *Cancer Res,* 01 April 2014, vol. 74 (7), 1902-12

- **BARBIERI, S. ; BRÖNNIMANN, M. ; BOXLER, S. et al.** Differentiation of prostate cancer lesions with high and with low Gleason score by diffusion-weighted MRI. *Eur Radiol,* 2016, 1-9
- **SUI, Y. ; H. WANG et al.** Differentiation of Low- and High-Grade Pediatric Brain Tumors with High b-Value Diffusion-weighted MR Imaging and a Fractional Order Calculus Model. *Radiology,* 2015, vol. 277 (2), 489-496
- Diffusion weighted imaging of prostate cancer: Prediction of cancer using texture features from parametric maps of the monoexponential and kurtosis functions. **MONTOYA, P.I. et al.** 2016 Sixth International Conference on Image Processing Theory, Tools and Applications (IPTA). IEEE, 2016, 1-6

- **EBERL MM ; FOX CH ; EDGE SB ; CARTER CA ; MAHONEY MC.** BI-RADS classification for management of abnormal mammograms. *J Am Board Fam Med.,* 2006, vol. 19 (2), 161-64
- **WEINREB JC ; BARENTSZ JO ; CHOYKE PL ; CORNUD F ; HAIDER MA ; MACURA KJ ; MARGOLIS D ; SCHNALL MD ; SHTERN F ; TEMPANY CM.** PI-RADS Prostate Imaging-Reporting and Data System:2015,Version 2. *Eur Urol,* 2016, vol. 69 (1), 16-40